# FASCICULE DE BREVET EUROPEEN

(11) **EP 1 537 117 B1**
(45) Date de publication et mention de la délivrance du brevet: **17.01.2007**
(21) Numéro de dépôt: 03769564.0
(22) Date de dépôt: 04.09.2003
(51) Int. Cl.: C07D 487/08, A61K 31/4995, A61P 31/00, C07D 237/28, C07D 243/02

(54) **DERIVES BENZOTRIAZABIBICYCLIQUES ET SON UTILISATION COMME INHIBITEURS DE BETA-LACTAMASES ET ANTI-BACTERIENS**
BENZOTRIAZABICYCLISCHE DERIVATE UND DEREN VERWENDUNG ALS BETA-LACTAMASEINHIBITOREN UND ANTIBAKTERIELLE MITTEL
BENZOTRIAZABICYCLIC DERIVATIVES AND USE THEREOF AS BETA-LACTAMASE INHIBITORS AND ANTI-BACTERIAL AGENTS

(30) Priorité: 05.09.2002 FR 0210957
(43) Date de publication de la demande: 08.06.2005
(73) Titulaire: Aventis Pharma S.A., 92160 Antony (FR)
(72) Inventeur: MUSICKI, Branislav, F-75019 Paris (FR)
(74) Mandataire: Hirsch & Associés
(86) Numéro de dépôt international: PCT/FR2003/002639
(87) Numéro de publication internationale: WO 2004/022563

(56) Documents cités:
- WO-A-02/10172
- WO-A-02/100860

## Description

L'invention concerne de nouveaux composés hétérocycliques, leur préparation et leur utilisation comme médicaments, notamment comme inhibiteurs de béta-lactamases et anti-bactériens.

Dans le journal J. Org. Chem., Vol. 37, No. 5, 1972, pages 697 à 699 est décrite notamment la préparation d'un dérivé bicyclique de formule brute C₁₀H₁₈N₂O.

Dans le journal J. Org. Chem., Vol. 45, No. 26, 1980, pages 5325-5326 est décrite notamment la préparation de dérivés bicycliques de formules brutes C₆H₉NO₂ et C₇H₁₁NO₂.

Dans la revue Chemical Reviews, 1983, vol. 83, No. 5, pages 549 à 555 est décrite notamment la préparation de dérivés bicycliques de fomules brutes C₁₀H₁₈N₂O et C₇H₁₂N₂O.

Dans le journal Angew. Chem. Int. Ed. 2000, 39, n° 3, pages 625 à 628 est décrit notamment la préparation d'un composé de formule brute C₁₂H₁₂N₂O.

Aucune utilisation particulière dans le domaine thérapeutique de ces composés n'a été décrite dans ces documents.

La demande WO-A-02/10172 décrit quant à elle des composés hétérocycliques, notamment de type 7-oxo-1-aza ou 1,6-diazabicyclo[3.2.1]octane diversement substitués, présentant des propriétés antibactériennes.

La présente invention a pour objet les composés répondant à la formule (I) suivante : 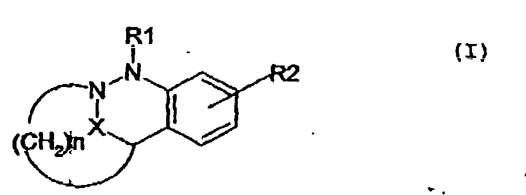 dans laquelle :
n est égal à 1 ou 2,
R₁ représente un atome d'hydrogène, un radical alkyle renfermant jusqu'à 8 atomes de carbone, un radical (CH₂)_{n'}R^{o}₁ dans lequel n' est égal à O ou 1 et R^{o}₁ représente un radical aryle renfermant jusqu'à 12 atomes de carbone, hétéroaryle renfermant jusqu'à 15 atomes de carbone et un ou plusieurs hétéroatomes choisis parmi les atomes d'azote, de soufre et d'oxygène, COR', CONR'R", CSNR'R", COCOOR', SO₂NR'R", SO₂R', CO₂R' ou CN,
R' représentant un atome d'hydrogène, un radical alkyle, alkényle renfermant jusqu'à 8 atomes de carbone, aralkyle renfermant jusqu'à 12 atomes de carbone ou aryle renfermant jusqu'à 12 atomes de carbone, R" représentant un atome d'hydrogène, un radical alkyle renfermant jusqu'à 8 atomes de carbone, aryl renfermant jusqu'à 12 atomes de carbone, aralkyle renfermant jusqu'à 12 atomes de carbone, SO₂-R' ou COR', R' étant défini comme ci-dessus,
R₂ représente un atome d'hydrogène ou un ou plusieurs substituants choisis dans le groupe constitué par les radicaux halogeno, alkyl, OH, Oalkyl, NO₂, NH₂, NHalkyl, N(alkyl)₂, NHCOalkyl, NHSO₂alkyl, CONHalkyl, SO₂NHalkyl, COOH, COOalkyl, CN, OSO₂alkyl, NHCONHalkyl et COalkyl, alkyl renfermant jusqu'à 8 atomes de carbone,
X représente un groupement divalent -C(O)-N(OR₃)- relié à l'atome d'azote par l'atome de carbone, dans lequel R₃ est choisi dans le groupe constitué par un atome d'hydrogène et les radicaux R, Y, Y₁, Y₂, et Y₃,
R est choisi dans le groupe constitué par un radical alkyle renfermant jusqu'à 6 atomes de carbone, éventuellement substitué par un radical pyridyle ou carbamoyle, un radical alkényle renfermant jusqu'à 8 atomes de carbone, aryle renfermant jusqu'à 12 atomes de carbone ou aralkyle renfermant jusqu'à 12 atomes de carbone, le noyau du radical aryle ou aralkyle étant éventuellement substitué par un radical OH, NH₂, NO₂, alkyle renfermant jusqu'à 8 atomes de carbone, alkoxy renfermant jusqu'à 8 atomes de carbone ou par un ou plusieurs atomes d'halogène,
Y est choisi dans le groupe constitué par les radicaux COR, COOH, COOR, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂,
Y₁ est choisi dans le groupe constitué par les radicaux SO₂R, SO₂NHCOR, SO₂NHCOOR, SO₂NHCONHR et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NR tétrazole, NR tétrazole substitué par le radical R et NRSO₂R, R étant défini comme ci-dessus.

L'invention a également pour objet les sels de ces composés qui peuvent être obtenus avec des bases ou des acides minéraux ou organiques.

L'atome de carbone asymétrique contenu dans les composés de formule (I) peut se présenter sous la configuration R, S ou RS et l'invention a donc également pour objet les composés de formule (I) se présentant sous la forme d'énantiomères purs ou sous la forme d'un mélange d'énantiomères notamment de racémates.

Par radical alkyle renfermant jusqu'à 8 atomes de carbone, on entend notamment le radical méthyle, éthyle, propyle, isopropyle, ainsi que butyle, pentyle ou hexyle linéaire ou ramifié.

Par radical alkényle renfermant jusqu'à 8 atomes de carbone, on entend par exemple le radical allyle, ou le radical butényle, pentényle ou hexényle.

Par radical aryle renfermant jusqu'à 12 atomes de carbone, on entend un radical phényle ou naphtyle.

Par radical aralkyle renfermant jusqu'à 12 atomes de carbone, on entend un radical benzyle, phénéthyle ou méthylnaphtyle.

Par radical alkoxy renfermant jusqu'à 8 atomes de carbone, on entend notamment le radical méthoxy, éthoxy, propoxy, isopropoxy, butoxy, isobutoxy, sec-butoxy ou tert-butoxy.

Par radical halogéno ou par atome d'halogène, on entend fluor, chlore, brome ou iode.

Par radical squarate, on entend le radical de formule :

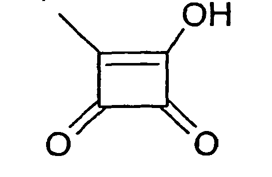

Par radical hétéroaryle, on entend notamment ceux choisis dans la liste qui suit. 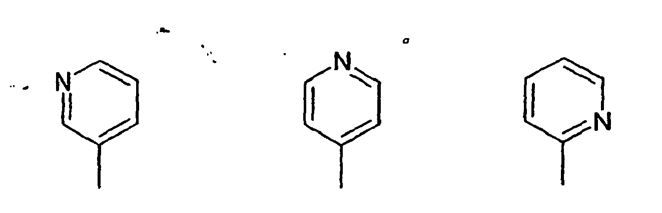 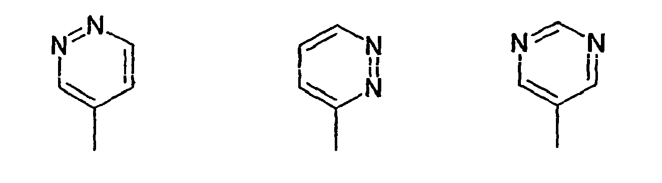 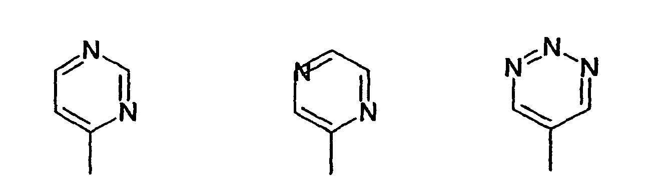 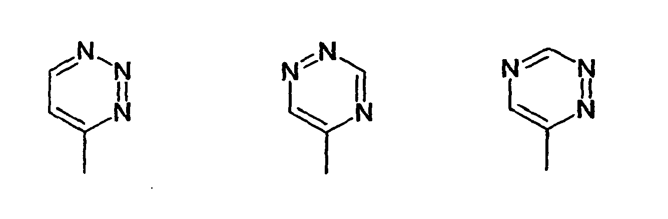 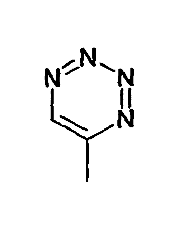 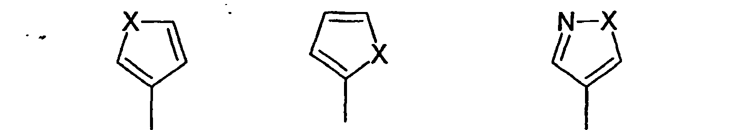 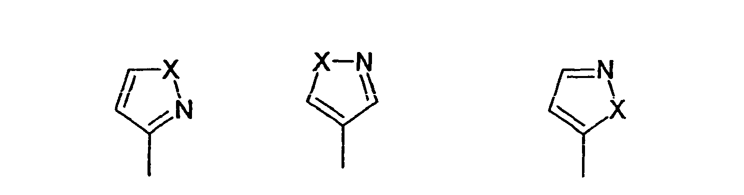 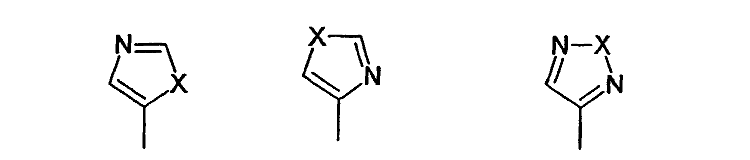 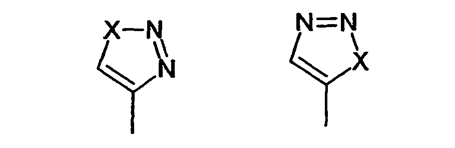 Avec X = S,O ou NR4 (R4 = H, alkyle)

Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkyl-phosphonium, les aryl-phosphoniums, les alkyl-aryl-phosphonium, les alkényl-aryl-phosphonium ou les sels d'ammoniums quaternaires tels que le sel de tétra-n-butyl-ammonium.

Parmi les composés de formule (I), l'invention a notamment pour objet ceux dans lesquels n est égal à 1, ceux dans lesquels R₂ est un atome d'hydrogène, ceux dans lesquels R₁ représente un atome d'hydrogène, un radical alkyle renfermant jusqu'à 8 atomes de carbone, un radical (CH₂)n'R^{o}₁ dans lequel n' est égal à O ou 1 et R^{o}₁ représente un radical aryle renfermant jusqu'à 12 atomes de carbone, hétéroaryle renfermant jusqu'à 15 atomes de carbone et un ou plusieurs hétéroatomes choisis parmi les atomes d'azote, de soufre et d'oxygène, CONR'R", CSNR'R", COCOOR' , SO₂NR'R", SO₂R' ou CO₂R', R' et R" étant définis comme précédemment ainsi que ceux dans lesquels X représente un groupement divalent -C(O)-N(OR₃)-dans lequel R3 est choisi dans le groupe constitué par un atome d'hydrogène et les radicaux R, Y et Y₁, R, Y et Y₁ étant définis comme précédemment.

Parmi les composés de formule (I), l'invention a tout particulièrement pour objet les composés dont les noms suivent :
- l'acide [[1,5-dihydro-1-(méthylsulfonyl)-3-oxo-2,5-méthano-2*H*-1,2,4-benzotriazépin-4(3*H*)-yl]oxy]-acétique,
- l'acide[[1-[(benzoylamino)carbonyl]-1,5-dihydro-3-oxo-2,5-méthano-2*H*-1,2,4-benzotriazépin-4(3*H*)-yl]oxy]-acétique,
- l'acide[[1,5-dihydro-3-oxo-1-[(phénylsulfonyl)amino carbonyl]-2,5-méthano-2*H*-1,2,4-benzotriazépin-4(3*H*)-yl]oxy]-acétique,
- l'acide [(1,5-dihydro-3-oxo-2,5-méthano-2*H*-1,2,4-benzotriazépin-4(3*H*)-yl)oxy]-acétique,
- la 4,5-dihydro-1-méthyl-4-(sulfooxy)-2,5-méthano-2*H-*1,2,4-benzotriazépin-3(1*H*)-one,
- la 4,5-dihydro-4-(2-propényloxy)-1-(3-pyridinylméthyl)-2,5-méthano-2*H*-1,2,4-benzotriazépin-3(1*H*)-one,
- le 4,5-dihydro-3-oxo-*N*-(phénylsulfonyl)-4-(2-propényloxy)-2,5-méthano-2*H*-1,2,4-benzotriazépine-1 (3*H*)-carboxamide,
- le N-benzoyl-4,5-dihydro-3-oxo-4-(2-propényloxy)-2,5-méthano-2*H*-1,2,4-benzotriazépine-1(3*H*)-carboxamide,
- le 4,5-dihydro-α,3-dioxo-4-(2-propényloxy)-2,5-méthano-2*H*-1,2,4-benzotriazépin-1(3*H*)-acétate d'éthyle,
- le 4,5-dihydro-3-oxo-4-(sulfooxy)-2,5-méthano-2*H-*1,2,4-benzotriazépin-1(3*H*)-acétate d'éthyle,
ainsi que leurs sels tels que définis plus haut.

Un autre objet de l'invention est un procédé permettant la préparation des composés de formule (I).

Ce procédé se caractérise en ce qu'il comporte :
a) une étape au cours de laquelle on fait réagir avec un agent de carbonylation, le cas échéant en présence d'une base, un composé de formule (II) : 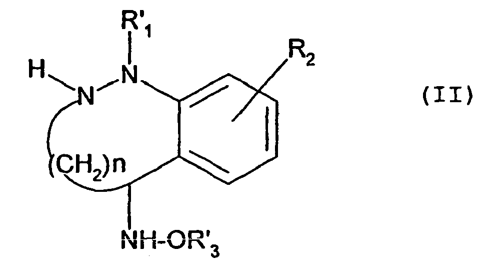 dans laquelle :
   R'₁ représente un radical R₁ ou un radical qui est un précurseur de R₁, R₂ et n sont tels que définis précédemment et R'₃ représente un groupement protecteur de l'hydroxy ou un radical Rp, Yp, Y₁p, Y₂p ou Y₃p, ces radicaux ayant respectivement les valeurs de R, Y, Y₁, Y₂ et Y₃ indiquées précédemment, dans lesquelles les éventuelles fonctions réactives présentes sont le cas échéant protégées, en vue d'obtenir un composé intermédiaire de formule (III) : 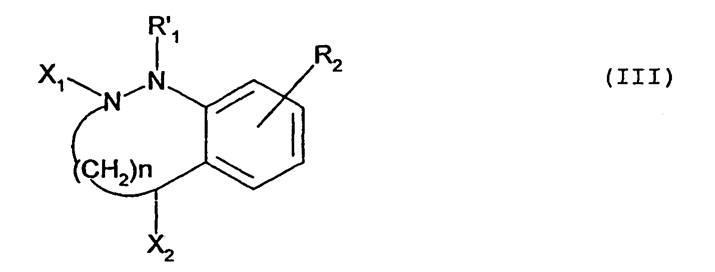
   dans laquelle :
   R'₁, R₂ et n ont les mêmes significations que ci-dessus et soit X₁ est un atome d'hydrogène et X₂ représente un groupement -N(OR'₃)-CO-X₃, R'₃ étant tel que défini précédemment et X₃ représentant le reste de l'agent de carbonylation, soit X₂ représente un groupement -NH-OR'₃ et X₁ représente un groupement CO-X₃, X₃ étant défini comme précédemment ;
b) une étape au cours de laquelle on cyclise l'intermédiaire obtenu précédemment, en présence d'une base ; et en ce que :
c) le cas échéant, l'étape a) est précédée et/ou l'étape b) est suivie de l'une ou de plusieurs des réactions suivantes, dans un ordre approprié :
   - protection des fonctions réactives,
   - déprotection des fonctions réactives,
   - estérification
   - saponification,
   - sulfatation,
   - phosphatation
   - amidification,
   - acylation,
   - sulfonylation ;
   - alkylation ;
   - formation d'un groupe urée ;
   - introduction d'un groupement tétrazole;
   - réduction d'acides carboxyliques ;
   - déshydratation d'amide en nitrile ;
   - salification ;
   - échange d'ions ;
   - séparation d'énantiomères ;
   - nitration ;
   - réduction d'un nitro en amino ;
   - halogénation ;
   - carbamoylation ;
   - cyanuration.

Comme agent de carbonylation, on peut mettre en oeuvre un réactif tel que le phosgène, le diphosgène, le triphosgène, un chloroformiate d'aryle tel que le chloroformiate de phényle ou de p-nitrophényle, un chloroformiate d'aralkyle tel que chloroformiate de benzyle, un chloroformiate d'alkyle ou d'alkényle tel que le chloroformiate de méthyle ou d'allyle, un dicarbonate d'alkyle tel que le dicarbonate de tert-butyle, le carbonyl-diimidazole et leurs mélanges.
La réaction a lieu de préférence en présence d'une base ou d'un mélange de bases qui neutralise l'acide formé. Elle peut notamment être une amine telle que la triethylamine, la diisopropyléthylamine, la pyridine, la diméthylaminopyridine. Toutefois, on peut également opérer en utilisant le produit de départ de formule II comme base. On en utilise alors un excès.

Le cas échéant, le produit de formule II est mis en oeuvre sous la forme d'un sel d'acide, par exemple un chlorhydrate ou un trifluoroacétate.

Comme base dans l'étape b), on peut également utiliser les amines, ou encore les hydrures, les alcoolates, les amidures ou carbonates de métaux alcalins ou alcalino-terreux.

Les amines peuvent être choisies par exemple dans la liste ci-dessus.

Comme hydrure on peut notamment utiliser l'hydrure de sodium ou de potassium. Comme alcoolate de métal alcalin, on utilise de préférence le t-butylate de potassium.

Comme amidure de métal alcalin on peut notamment utiliser le bis(triméthylsilyl) amidure de lithium.

Comme carbonate, on peut notamment utiliser le carbonate ou bicarbonate de sodium ou de potassium.

Le cas échéant, l'intermédiaire de formule III peut être obtenu sous forme d'un sel d'acide généré lors de la réaction de carbonylation et notamment un chlorhydrate. Il est ensuite mis en oeuvre dans la réaction de cyclisation sous cette forme.

Le cas échéant, la cyclisation peut être effectuée sans isolement de l'intermédiaire de formule III.

Les réactions mentionnées à l'étape c) sont d'une manière générale des réactions classiques, bien connues de l'homme du métier.Des illustrations sont fournies ci-après dans la partie expérimentale.

Les fonctions réactives qu'il convient, le cas échéant, de protéger sont les fonctions acides carboxyliques, amines, amides, hydroxy et hydroxylamines.

La protection de la fonction acide est notamment effectuée sous forme d'esters d'alkyle, d'esters allyliques, de benzyle, benzhydryle ou p-nitrobenzyle.

La déprotection est effectuée par saponification, hydrolyse acide, hydrogénolyse, ou encore clivage à l'aide de complexes solubles du Palladium O.

La protection des amines, des azotes hétérocycliques et des amides est notamment effectuée, selon les cas, sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényl tertbutyl-silyle, ou de dérivés phénylsulfonylalkyle ou cyanoalkyle.

La déprotection est effectuée, selon la nature du groupement protecteur, par le sodium ou le lithium dans l'ammoniac liquide, par hydrogénolyse ou à l'aide de complexes solubles du Palladium O, par action d'un acide, ou par action du fluorure de tétrabutylammonium ou de bases fortes telles que l'hydrure de sodium ou le t.butylate de potassium.

La protection des hydroxylamines est effectuée notamment sous forme d'éthers de benzyle ou d'allyle.

Le clivage des éthers est effectué par hydrogénolyse ou à l'aide de complexes solubles du Palladium O.

La protection des alcools et des phénols est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

La réaction de sulfatation est effectuée par action des complexes SO₃-amines tels que SO₃-pyridine ou SO₃-diméthylformamide, en opérant dans la pyridine, le sel formé, par exemple le sel de pyridine, pouvant ensuite être échangé par exemple par un sel d'une autre amine, d'un ammonium quaternaire ou d'un métal alcalin.

La réaction de phosphatation est effectuée par exemple par action d'un chlorophosphate tel que le diméthyl, dibenzyl ou diphényl chlorophosphate.

La réaction d'amidification est effectuée au départ de l'acide carboxylique à l'aide d'un agent d'activation tel qu'un chloroformiate d'alkyle, l'EDCI (1-(3-diméthylamino-propyl)-3-éthylcarbo-diimide chlorhydrate) ou le BOP(benzotriazol-1-yloxytripyrolidino-phosphonium hexafluorophosphate) par action de l'ammoniaque ou d'une amine appropriée ou de leurs sels d'acides.

Les réactions d'acylation" et de sulfonylation sont effectuées sur les hydroxyurées, les alcools, les amines ou les azotes hétérocycliques, par action selon les cas, d'un halogènure ou d'un anhydride d'acide carboxylique ou d'acide sulfonique approprié, le cas échéant en présence d'une base.

La réaction d'alkylation est effectuée par action sur les dérivés hydroxylés, les énolates d'esters ou de cétones, les amines ou les azotes hétérocycliques, selon les cas, d'un sulfate d'alkyle ou d'un halogènure d'alkyle ou d'alkyle substitué, notamment par un radical carboxy libre ou estérifié.

La réduction d'acides en alcools peut être effectuée par action d'un borane ou via un anhydride mixte intermédiaire, par action d'un borohydrure alcalin. L'anhydride mixte est préparé par exemple à l'aide d'un chloroformiate d'alkyle. La réduction d'aldéhyde en alcool est de préférence effectuée par action de borohydrure de sodium.

La déshydratation d'amide en nitrile peut intervenir dans les conditions des réactions de carbonylation et cyclisation.

La salification par les acides est le cas échéant réalisée par addition d'un acide en phase soluble au composé. La salification par les bases peut concerner les composés comportant une fonction acide et notamment les composés comportant une fonction carboxy, ceux comportant une fonction sulfooxy ou dérivée de l'acide phosphorique ou ceux comportant un hétérocycle à caractère acide.

Dans le cas d'une fonction carboxy, on opère par addition d'une base appropriée telle que celles citées précédemment. Dans le cas d'une fonction sulfooxy ou dérivée de l'acide phosphorique, on obtient directement le sel de pyridinium lors de l'action du complexe SO3-pyridine et on obtient les autres sels à partir de ce sel de pyridinium. Dans l'un ou l'autre cas, on peut encore opérer par échange d'ions sur résine.

La nitration peut être effectuée par l'acide nitrique ou par l'un de ses sels métalliques, en milieu acide.

La réduction d'un groupement nitro peut être effectuée par le dithionite de sodium ou encore par le zinc dans l'acide acétique.

Par halogénation on entend introduction d'un substituant halogéné à partir d'un hydroxy ou halogénation directe du cycle aromatique. Selon le cas , la réaction peut par exemple être mise en oeuvre par action d'iode ou en présence de triphénylphosphine, par action de brome dans l'acide acétique ou encore d'iode en présence de C₆H₅I(OCOCF₃)₂, ou encore par réaction d'un réactif halogéné électrophile tel que le N-fluorosulfonylimide en présence d'une base forte. De tels réactifs sont connus de l'homme du métier.

La réaction de carbamoylation peut être réalisée par la mis en oeuvre d'un chloroformiate puis d'une amine ou, le cas échéant, d'ammoniac.

L'introduction d'un cyano est réalisée par substitution nucléophile à l'aide d'un cyanure alcalin ou du bromure de cyanogène.

La séparation des énantiomères peut être réalisée selon les techniques connues de l'homme du métier, notamment la chromatographie.

Outre via les procédés décrits précédemment, des composés de formule (I) peuvent bien entendu être obtenus par des méthodes qui utilisent au départ un composé de formule (II) dans laquelle R'₁, R₂, et R'₃ ont les valeurs qui conduisent directement (sans transformation) à celles des composés que l'on souhaite préparer. Le cas échéant, celles de ces valeurs qui renfermeraient des fonctions réactives telles que mentionnées plus haut sont alors protégées, la déprotection intervenant à l'issue de l'étape de cyclisation b ou à tout autre moment opportun dans la synthèse. Les protections et déprotections sont alors réalisées comme décrit ci-dessus.

L'invention a encore pour objet un procédé selon ce qui précède, caractérisé en ce que le composé de formule (II) est obtenu par un procédé selon lequel on traite un composé de formule (IV) : 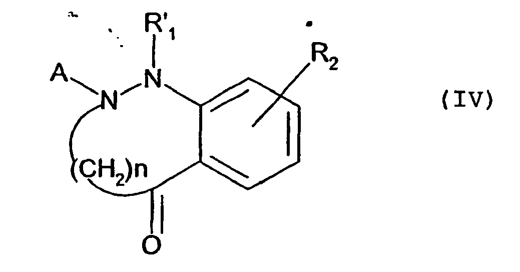 dans laquelle R'₁, R₂ et n sont définis comme précédemment, et A représente un atome d'hydrogène ou un groupement protecteur de l'azote, par un agent de réduction, pour obtenir un composé de formule (V) : 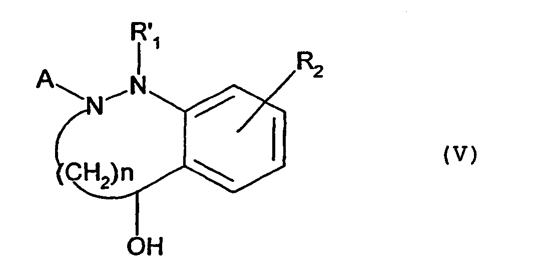 dans laquelle A, R'₁, R₂ et n conservent leur signification précitée, dans lequel, le cas échéant, l'on remplace le groupement OH par un groupe partant, pour obtenir un composé de formule (VI) : 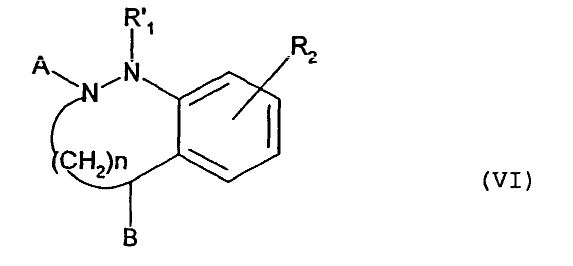 dans laquelle A, R'₁, R₂, et n conservent leur signification précitée et B représente un groupe partant, que l'on traite par un composé de formule NH₂-OR'₃, R'₃ conservant la signification précitée, puis, le cas échéant, par un agent de déprotection approprié de l'atome d'azote.

L'invention a encore pour objet un procédé selon ce qui précède, caractérisé en ce que le composé de formule (II) est obtenu par un procédé selon lequel on traite un composé de formule (IV) telle que définie précédemment, par un composé de formule H₂N-OR'₃, pour obtenir un composé de formule (VII) : 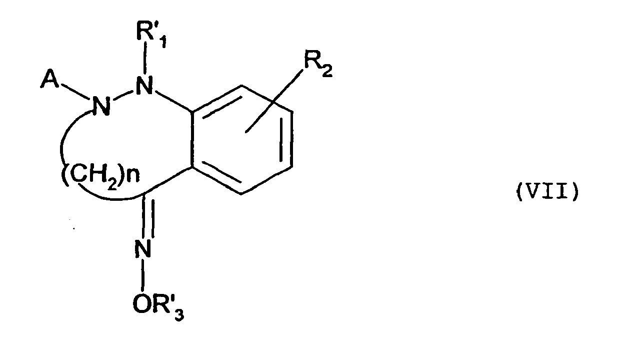 dans laquelle A, R'₁, R₂, n et R'₃ sont définis comme précédemment, que l'on fait réagir avec un agent de réduction pour obtenir un composé de formule (VIII) : 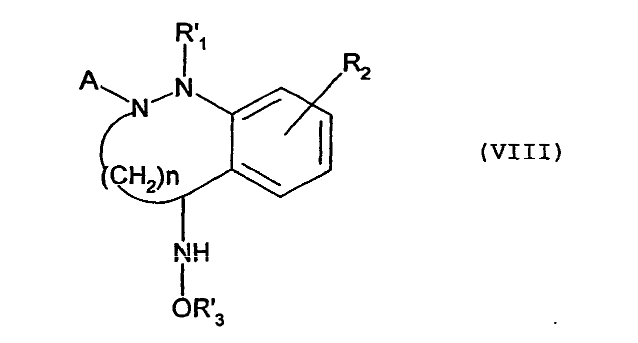 dans laquelle A, R'₁, R₂, n et R'₃ sont définis comme précédemment, que l'on traite, le cas échéant, par un agent de déprotection de l'atome d'azote approprié.

Le groupement protecteur de l'azote est notamment l'un de ceux qui sont cités plus haut.

L'agent de réduction est notamment un borohydrure alcalin.

Le groupe partant est notamment un sulfonate, par exemple un mésylate ou un tosylate, obtenu par action de chlorure de sulfonyle correspondant en présence d'une base, ou un halogène, plus particulièrement un chlore, un brome ou un iode, obtenu par exemple par action du chlorure de thionyle ou de P(C₆H₅)₃CBr₄ ou PBr₃ ou, dans le cas d'un atome d'iode, par action d'un iodure alcalin sur un sulfonate.

L'agent de déprotection est notamment l'un de ceux mentionnés plus haut.

L'agent de réduction que l'on fait agir sur le composé de formule (VII) est notamment le cyano ou l'acétoxyborohydrure de sodium.

Les composés de formule générale (I) possèdent une bonne activité antibiotique sur les bactéries gram(+) telles que les staphylocoques. Leur efficacité sur les bactéries gram (-) notamment sur les entérobactéries est particulièrement notable.

Ces propriétés rendent aptes lesdits produits ainsi que leurs sels d'acides et de bases pharmaceutiquement acceptables à être utilisés comme médicaments dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

Les composés de formule générales (I) sont par ailleurs doués de propriétés inhibitrices de béta-lactamases, et présentent par là de l'intérêt dans la lutte contre les maladies infectieuses ou la prévention de celles-ci, sous forme d'association avec divers composés antibiotiques de type β-lactamines, afin de renforcer leur efficacité dans la lutte contre les bactéries pathogènes productrices de β-lactamases.

Il est bien connu que l'inactivation enzymatique des antibiotiques de type β-lactamines, que ce soit des composés de type pénicillines ou céphalosporines, dans le traitement des infections bactériennes est un obstacle pour ce type de composés. Cette inactivation consiste en un processus de dégradation des β-lactamines et constitue l'un des mécanismes par lesquels les bactéries peuvent devenir résistantes aux traitements. Il est donc souhaitable de parvenir à contrer ce processus enzymatique en associant à l'agent antibactérien de type β-lactamines un agent susceptible d'inhiber l'enzyme. Lorsqu'un inhibiteur de β-lactamase est utilisé en combinaison avec un antibiotique de type β-lactamines, il peut donc renforcer son efficacité contre certains microorganismes.

La présente invention a donc également pour objet, à titre de médicaments et notamment de médicaments destinés au traitement des infections bactériennes chez l'homme ou l'animal et de médicaments destinés à inhiber la production des β-lactamases pour les bactéries pathogènes, les composés de formule (I) tels que définis ci-dessus ainsi que leurs sels avec les acides et les bases pharmaceutiquement acceptables, et notamment les composés préférés mentionnés plus haut.

L'antibiotique de type β-lactamines auquel peut-être associé le composé de formule (I) peut être choisi dans le groupe constitué par les pénames, les pénèmes, les carbapénèmes, les céphèmes, les carbacéphèmes, les oxacéphèmes, les céphamycines et les monobactames.

Par β-lactamines, on entend par exemple les pénicillines telles que amoxicilline, ampicilline, azlocilline, mezlocilline, apalcilline, hetacilline, bacampicilline, carbenicilline, sulbenicilline, ticarcilline, piperacilline, azlocilline, mecillinam, pivmecillinam, methicilline, ciclacilline, talampicilline, aspoxicilline, oxacilline, cloxacilline, dicloxacilline, flucloxacilline, nafcilline ou pivampicilline, les céphalosporines telles que céphalothine, céphaloridine, céfaclor, céfadroxile, céfamandole, céfazoline, céphalexine, céphradine, ceftizoxime, céfoxitine, céphacétrile, céfotiam, céfotaxime, cefsulodine, céfopérazone, ceftizoxime, cefménoxime, cefmétazole, céphaloglycine, céfonicide, céfodizime, cefpirome, ceftazidime, ceftriaxone, cefpiramide, cefbupérazone, cefozopran, céfépime, céfoselis, céfluprenam, céfuzonam, cefpimizole, cefclidine, céfixime, ceftibutène, cefdinir, cefpodoxime axétil, cefpodoxime proxétil, ceftéram pivoxil, céfétamet pivoxil, cefcapène pivoxil ou cefditoren pivoxil, céfuroxime, céfuroxime axétil, loracarbacef, latamoxef, les carbapénèmes tels que imipénème, méropénème, biapénème ou panipénème et les monobactames tels que l'aztréonam et le carumonam, ainsi que leur sels.

Les composés de formule (I) ou leurs sels pharmaceutiquement acceptables, peuvent être administrés en même temps que la prise d'antibiotiques de type β-lactamines, ou séparément, de préférence après celle-ci. Cela peut s'effectuer sous forme d'un mélange des deux principes actifs ou sous forme d'une association pharmaceutique des deux principes actifs séparés.

La posologie des composés de formule (I) et de leurs sels pharmaceutiquement acceptables peut bien entendu varier dans de larges limites et doit naturellement être adaptée, dans chaque cas particulier, aux conditions individuelles et à l'agent pathogène à combattre. En général, pour une utilisation dans la traitement des infections bactériennes, la dose journalière peut être comprise entre 0,250 g et 10 g par jour, par voie orale chez l'homme, avec le produit décrit à l'exemple 11 ou encore comprise entre 0,25 g et 10 g par jour par voie intramusculaire ou intraveineuse. Pour une utilisation comme inhibiteur de β-lactamase, une dose journalière chez l'homme pouvant aller de 0,1 à environ 10 g peut convenir.

Par ailleurs, le rapport de l'inhibiteur de β-lactamase de formule (I) ou du sel pharmaceutiquement acceptable de celui-ci à l'antibiotique de type β-lactamines peut également varier dans de larges limites et doit être adapté, dans chaque cas particulier, aux conditions individuelles. En général, un rapport allant d'environ 1:20 à environ 1:1 devrait être indiqué.

Les médicaments antibiotiques ou inhibiteurs de β-lactamases tels que définis plus haut sont mis en oeuvre sous forme de compositions pharmaceutiques en mélange avec un excipient pharmaceutique inerte, organique ou minéral, adapté au mode d'administration recherché, et l'invention a également pour objet les compositions pharmaceutiques renfermant comme principe actif, au moins un des composés de l'invention tels que définis plus haut.

Ces compositions peuvent être administrées par voie buccale, rectale, parentérale, notamment intramusculaire, ou par voie locale en application topique sur la peau et les muqueuses.

Ces compositions peuvent être solides ou liquides et se présentent sous les formes pharmaceutiques couramment utilisées en médecine humaine, comme par exemple les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, telles que le talc, la gomme arabique,le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants et les conservateurs.

Ces compositions peuvent également se présenter sous forme de lyophilisat destiné à être dissout extemporanément dans un véhicule approprié par exemple de l'eau stérile apyrogène.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I) :
- les produits de formule (III) telle que définie précédemment ainsi que leurs sels avec les acides et notamment leurs chlorhydrates et trifluoroacétates,
- les produits de formule (II) telle que définie précédemment ainsi que leurs sels avec les acides et notamment leurs chlorhydrates et trifluoroacétates, ainsi que les produits de formules (IV), (V), (VI), (VII) et (VIII) telles que définies précédemment, ainsi que leurs sels avec un acide et notamment leurs chlorhydrates et trifluoroacétates.

Les produits de formule (IV) sont préparables par exemple selon des méthodes fournies ci-après dans la partie expérimentale.

Les exemples suivants illustrent l'invention, sans toutefois en limiter la portée.

### Exemple 1 : 4-(2-propényloxy)-2,3,4,5-tétrahydro-2,5-méthano-1H-1,2,4-benzotriazépin-3-one

### Stade A : chlorhydrate de 4-cinnoléinol

Dans un réacteur, on introduit 560 ml d'acide chlorhydrique concentré. 111,6 g de 2-acétyl amino phényle sont ajoutés à température ambiante. A -5°C, on additionne à cette solution orangée, en 1 heure, 62,8 mg de nitrite de sodium en solution dans 170 mL d'eau. La température est maintenue en dessous de 0°C pendant toute l'introduction. Le milieu réactionnel est chauffé à 65°C pendant 3 heures. On refoidit ensuite pendant 20 minutes, puis on essore et lave à l'éther. Le composé est sèché sur P₂O₅ pendant une nuit à 45°C. On obtient 118.6 grammes (**77%**) de produit attendu. 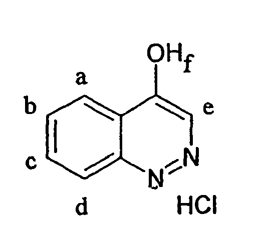
Spectre RMN : (DMSO) 1H : 7.43 ppm (tl,J = 7,5) 1H : 7.80 ppm (td,J = 7,5 et 1,5) Hb et Hc ; 1H : 7.68 ppm (dl,J = 7,5) 1H : 8.04 ppm (dl,J = 7,5) Ha et Hd ; 1H : 7.76 ppm (s)He ; 1H : 13.8 ppm (s) OH
Spectre de Masse : 146+ M+
36+/38+ Doublet caractéristique H³⁵Cl⁺/H³⁷Cl⁺
Spectre IR : 1625/1564 cm-1 Système conjugué + aromatique
Spectre UV : 242 nm ε=8700
   340 nm ε=6700

### Stade B : chlorhydrate de 2,3-dihydro-4(1H)-cinnoléinone

69,41 g de produit obtenu au stade A sont solubilisés dans 2,5 l d'éthanol. On ajoute ensuite 62,79 g de zinc en poudre, puis lentement, un mélange de 300 ml d'éthanol et 150 ml d'acide acétique à température ambiante. On chauffe à reflux pendant 30 mn. Le milieu réactionnel est ensuite décanté, et le résidu de zinc est lavé plusieurs fois à l'éthanol. On laisse refroidir 20 minutes dans un mélange glace-méthanol (-15°C), puis on ajoute une solution d'acide chlorhydrique gazeux dans l'acétate d'éthyle (350 mL ; 4M). Le précipité formé est essoré, lavé à l'éther, puis au pentane et enfin séché (sous pression réduite). On obtient 42.12 grammes (**60%**) de produit attendu. 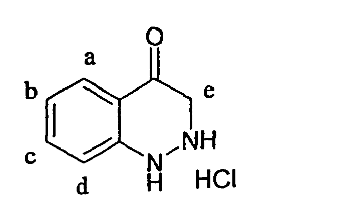
Spectre RMN : (DMSO) 2H : 4.04 ppm (s) He ; 1H : 7,00 ppm (td,J = 8-1,5) 1H : 7.55 ppm (td, J = 8-1,5) Hb et Hc ; 1H : 7.06 ppm (dl,J = 8) 1H : 7.73 ppm (dd,J = 8-1,5) Ha et Hd ; 1H : 9.77 ppm (s)Proton mobile
Spectre de Masse : 148+ M+ ; 119+ M+ ; 92+ M+ ; 36+/38+ salification du produit
Spectre IR : 1686 cm-1 v(C=O) ; 1606-1550-1520 cm-1 Système conjugué + aromatique

### Stade C : 3,4-dihydro-4-oxo-2(1H)-cinnoléinecarboxylate de 1,1-diméthyléthyle

On solubilise 82,16 g de produit obtenu au stade B dans le THF (1.7 L). On ajoute ensuite 106,72 g de (1,1-diméthyléthoxy)carbonyl-carbonate de 1,1-diméthyléthyle puis, pendant 15 mn, goutte à goutte, 94,4 mg de triéthylamine. On laisse sous agitation 20 heures puis on filtre pour éliminer les sels de triéthylamine que l'on rince au THF. On évapore le solvant et reprend avec un mélange heptane-AcOEt (1:2) et NaH₂PO₄ (solution aqueuse à 1M). On extrait à l'acétate d'éthyle et lave à l'eau. La phase organique est séchée sur MgSO₄ puis évaporée à sec. On obtient 65.42 grammes (59%)de produit attendu. 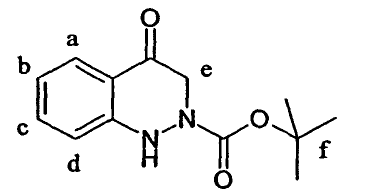
Spectre RMN : (CDCl3) 9H : 1.46 ppm (s) Hf ; 2H : 4.38 ppm (s) He ; 1H : 6.91 ppm (dl,J = 8) Hd ou Ha ; 1H : 6.96 ppm (td,J = 8-1,5) Hc ; 1H : 7.43 ppm (td,J = 8-1,5) Hb ; 1H : 7.91 ppm (dd,J = 8-1,5) Ha ou Hd ; 1H : 7.1 ppm (s) Proton mobile.
Spectre de Masse : 248+ M+ ; 233+ M+ -CH₃ ; 192+ M+ -tBu ; 148+ M+ -boc ; 119+ M+ -[-(NH-Nboc)-] ; 57+ tBu+ ;
Spectre IR : 1712-1670 cm-1 ν(C=O) ; 1610-1578 cm-1 ν(C=C) aromatique.

### Stade D : 3,4-dihydro-4-[(2-propényloxy)imino]-2(1H)-cinnoléinecarboxylate de 1,1-diméthyléthyle

30,8 g de produit obtenu au stade C sont solubilisés dans 200 ml de pyridine. Sous agitation et sous argon, 14,95 g d'alkyl hydroxylamine sont ajoutés. Au bout d'une heure. On évapore la pyridine. Le résidu est repris avec un mélange heptane-AcOEt (1:2) et NaHSO₄ (solution aqueuse à 10%). On extrait à l'acétate d'éthyle et lave à l'eau. La phase organique est séchée sur MgSO₄ et évaporée à sec. On isole 36.08 g de produit attendu (**96%**). 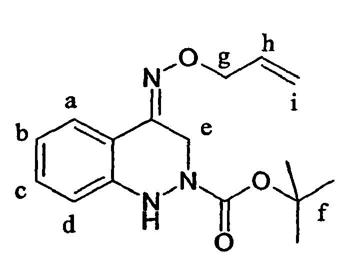
Spectre RMN : (CDC13) 9H : 1.44 ppm (s) Hf ; 2H : 4.73 ppm (s) He ; 2H : 4.69 ppm (dt, J = 5,5-1) Hg ; 1H : 5.22 ppm (dq,J = 10-1); Hi1 ; 1H : 5.32 ppm (dq,J = 17,5-1) H12 ; 1H : 6.05 ppm (m)Hh ; 1H : 6.91 ppm (td, J = 8-1,5) ; 1H : 7.22 ppm (td,J = 8-1,5) Hb et Hc ; 1H : 6.81 ppm (dl,J = 8) 1H : 7.86 ppm (dd,J = 8-1,5) Ha et Hd
Spectre de Masse : 304+ MH+ ; 247+ M+-(O-CH2-CH=CH2).
Spectre IR : 1708 cm-1 ν(C=O) ; 1638-1610-1589-1494 cm-1 Système conjugué + aromatique

### Stade E : 3,4-dihydro-4-[(2-propényloxy)amino]-2(1H)-cinnoléinecarboxylate de 1,1-diméthyléthyle

19 g de produit obtenu au stade D sont solubilisés dans 2 1 de méthanol, puis on ajoute 63,18 g de cyanoborohydrure de sodium. A 0°C, 107,03 g (95,5 ml) d'éthérate de trifluorure de bore sont introduits goutte à goutte. Après évaporation du méthanol, le résidu est traité par NaH₂PO₄ (solution aqueuse 1M), puis on extrait avec un mélange heptane-AcOEt (1:2). On lave ensuite à l'eau, sèche la phase organique avec MgSO₄ et évaporée à sec. Le composé est repris à 0°C avec un mélange éther/pentane. Le composé cristallise. On isole 13.95 g de produit attendu (73 %). 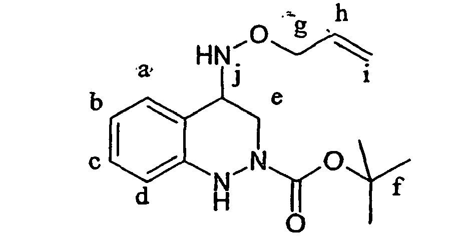
Spectre RMN :(CDCl3): 9H : 1.49 ppm (s) Hf ; 1H : 3.35 ppm (d) Hel ; 1H : 4.60 ppm (dd) He2 ; 1H : 4.15 ppm (t) Hj ; 1H : 4.30 ppm (m) Hg ; 1H : 5.20 ppm (m) Hi1 ; 1H : 5.30 ppm (m) Hi2 ; 1H : 5.96 ppm (m) Hb ; 1H : 6.75 ppm Hh ; 1H : 6.86 ppm Hd ; 1H : 7.16 ppm Hc ; 1H : 7.28 ppm Ha.
Spectre de Masse : 305+ %+ ; 205+ M+-CO2tBu+H ; 57+ tBu+.
Spectre IR : 3344 cm-1 ν(NH) ; 1708 cm-1 ν(C=O) ; 1638-1610-1589-1494 cm-1 ν(C=C) + aromatique.
Spectre UV : 244 nm ε=8500 ; 290 nm ε=2000.
Microanalyse :

| Calculé : | | Obtenu : | |
|---|---|---|---|
| %C : | 62.9% | %C : | 63% |
| %H : | 7.5% | %H : | 7.6% |
| %N : | 13.8% | %N : | 13.7% |

### Stade F : dichlorhydrate de 1,2,3,4-tétrahydro-4-[(2-propényloxy)amino]-cinnoléine

11,28 g de produit obtenu au stade E sont solubilisés dans 43 ml d'acétate d'éthyle, puis on ajoute 70 ml d'une solution d'acide chlorhydrique gazeux dans l'acétate d'éthyle à 5.3M à 0°C, sous agitation et sous argon. Au bout de 30 mn, le précipité est filtré, lavé à l'éther puis séché. On isole 8.93 g de composé attendu (100 %). 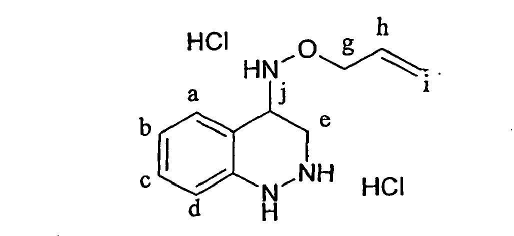
Spectre RMN : (DMSO) : 1H : 3,37 ppm (dd,J = 4-13) He₁ ; 1H : 3,68 ppm (dd, J) = 3-13) He₂ ; 2H : 4,23 ppm (dt, J = 5,5-1) Hg ; 1H : 4,28 (dd, J = 3-4) Hj ; 1H : 5,18 ppm (dq, J = 10,5-1,5) Hi1 ; 1H : 5,29 ppm (dq, J = 17,5-1,5) Hi2 ; 1H : 5,95 ppm (m) Hh ; 1H : 6,83 ppm (dd,J = 7,5-1), 1H : 7,39 ppm (dd,J = 7,5-1) Ha et Hd ; 1H : 6,93 (td, J = 7,5-1) ; 1H : 7,21 ppm (td,J = 7,5-1) Hb et Hc ; 1H : 7,32 (sl) proton mobile ; 1H : 8,96 (sl) proton mobile ; 1H : 11,00 (sl) proton mobile ; 1H : 11,78 (sl) proton mobile.
Spectre SM : 205+ M+ ; 36+/38+ H35Cl+/H37Cl+.
Spectre IR : >3000 cm-1 ν(NH) ; 1642 cm-1 ν(C=C); 1612-1590-1530-1497 cm-1 ν(C=C) + aromatique ;
Microanalyse :

| Calculé (avec deux chlorhydrates ): | | Obtenu : | |
|---|---|---|---|
| %C : | 47.5 % | %C : | 47.8% |
| %H : | 6.2% | %H : | 6.1% |
| %N : | 15.1% | %N : | 15.2% |
| %Cl : | 25.5% | %Cl : | 24.7% |

### Stade G : 4,5-dihydro-4-(2-propényloxy)-2,5-méthano-2H-1,2,4-benzotriazépin-3(1H)-one

8,93 g de produit obtenu au stade F sont solubilisés dans 3,7 1 d'acétonitrile. On ajoute goutte à goutte 14,92 g (20,6 ml) de triéthylamine. On introduit ensuite 3,66 g (2,25 ml) de diphosgène en 5 mn à O°C, puis 4,96 g de diméthylaminopyridine. On laisse ensuite remonter à température ambiante. Après une heure, on évapore l'acétonitrile et le résidu est traité par NaH₂PO₄ (solution acqueuse 1M). On extrait avec un mélange heptane-AcOEt (1:2), et on lave à l'eau. La phase organique est séchée sur MgSO₄. On filtre, on évapore, et le composé est repris dans l'éther à O°C, il cristallise. On obtient 3,94 g de composé attendu (**46%**). 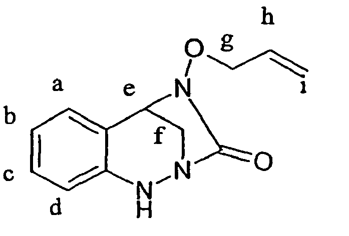
Spectre RMN : (CDCl3) : 1H : 3.29 ppm (d,J = 11,5) Hfl, 1H : 3.70 ppm (dd,J = 11,5-3) Hf2 ; 1H : 4.38 ppm (d,J = 3) He ; 2H : 4.42 ppm (dl,J = 6) Hg ; 1H : 6.02 ppm (m) Hh ; 1H : 5.33 ppm (dl, J = 10,5) Hi1 ; 1H : 5.38 ppm (dl,J = 17) Hi2 ; 1H : 6.63 ppm (dd,J = 8-1); 1H : 7.10 ppm (dd,J = 8-1,5) Ha et Hd ; 1H : 6.82 ppm (td,J = 8-1), 1H : 7.21 ppm (td,J = 8-1,5) Hc et Hb.
Spectre de Masse : 231+ M+ ; 174+ M+-(O-CH2-CH=CH2); 131+ Ouverture du cycle carbamate.
Spectre IR : 3312 cm-1 ν(NH) ; 1744 cm-1 ν(C=O) ; 1648 ν(C=C); 1608-1582-1492 cm-1 Aromatiques.
Spectre UV : 246 nm ε=7400 ; 291 nm ε=1800
Microanalyse :

| Calculé : | | Obtenu : | |
|---|---|---|---|
| %C : | 62.3% | %C : | 62.1% |
| %H : | 5.7% | %H : | 5.5% |
| %N : | 18.2% | %N : | 18.1% |

### Exemple 2 : 4-benzyloxy-2,3,4,5-tétrahydro-2,5-méthano-1H-1,2,4-benzotriazépin-3-one

### Stade A : 3,4-dihydro-4-[phénylméthoxy)imino-2(1H)-cinnoléinecarboxylate de 1,1-diméthyléthyle

3 g de composé obtenu au stade C de l'exemple 1 sont solubilisés dans 25 ml de pyridine, puis, sous agitation et sous argon, 2.12 g de chlorhydrate de benzylhydroxylamine sont ajoutés. Au bout d'une heure, on évapore la pyridine. Le résidu est repris avec un mélange heptane:AcOEt 1:2 et NaHSO₄ (solution à 10% dans H₂O). On extrait à l'acétate d'éthyle et lave à l'eau. La phase organique est séchée sur MgSO₄. On filtre, évapore le solvant et isole 4.3 g de composé attendu (100 %).

### Stade B : 3,4-dihydro-4-[(phénylméthoxy)-amino]-2(1H)-cinnoléinecarboxylate de 1,1-diméthyléthyle

4.27 g de composé obtenu au stade A sont solubilisés dans 450 ml de méthanol. On ajoute ensuite 12,14 g de cyanoborohydrure de sodium, puis, goutte à goutte, à 0°C, 20,57 g (18,36 ml) d'étherate de trifluorure de bore. Après évaporation du méthanol, le résidu est traité par NaH2PO4 (solution aqueuse 1M), puis on extrait avec un mélange heptane:AcOEt 1:2. On lave ensuite à l'eau acidifiée, puis sèche la phase organique avec MgSO4 et évapore le solvant. Le composé est repris à 0°C avec un mélange éther/pentane. Le composé cristallise. On isole 3.81 g de composé attendu (89%). 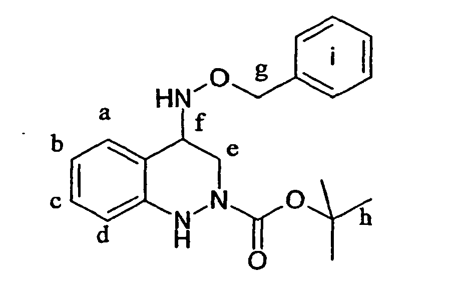
Spectre RMN : (CDCl3) 9H : 1.51 ppm (s) Hf ; 1H : 3.35 ppm (dl,J = 13,5) He1 ; 1H : 4.63 ppm (dl,J = 13,5) He2 ; 1H : 4.10 ppm (t,J = 2)Hf ; 2H : 4.81 ppm système de type AB Hg ; 1H : 6.75 ppm (d,J = 8), 1H : 6.83 ppm (td,J = 8-1,5), 2H : 7.16 ppm (m): Ha, b, c, d ; 5H : 7.26 à 7.42 ppm Hi.
Spectre de Masse : 356+ MH+ ; 378+ Mna+ ; 733+ [2M+Na]+ ; 300+ MH+-tBu ; 233+ MH+-(-NH-O-CH2-Ph) ; 177+ 233+-tBu ; 133+ 233+-CO2tBu ; 106+ (Ph-CH2-O)+.

### Stade C : dichlorhydrate de 1,2,3,4-tétrahydro-4-[(phénylméthoxy)amino]-cinnoléine

3.81 g de composé obtenu au stade B sont solubilisés dans 15 ml d'acétate d'éthyle, puis on ajoute 25 mL d'une solution d'acide chlorhydrique gazeux dans l'acétate d'éthyle à 4.3M à 0°C, sous agitation et sous argon. Au bout de 30 mn, le milieu réactionnel est filtré et lavé à l'éther. On sèche le composé et isole 3.13 g de composé attendu. (**89%**).

### Stade D : 4,5-dihydro-4-(phénylméthoxy)-2,5-méthano-2H-1,2,4-benzotriazépin-3(1H)-one

3,13 g de composé obtenu au stade C sont solubilisés dans 1,9 1 d'acétonitrile. On ajoute goutte à goutte en 10 minutes 4,81 g (6,6 ml) de triéthylamine. On ajoute ensuite lentement à 0°C, 0,943 g (575µl) de diphosgène, puis 1,27 g de diméthyl amino pyridine. On laisse ensuite remonter à température ambiante. Après une heure, on évapore l'acétonitrile et le résidu est traité par NaH₂PO₄ (solution aqueuse 1M). On extrait avec un mélange heptane:AcOEt 1:2, et on lave à l'eau. La phase organique est séchée sur MgSO₄ et évaporée à sec. Ce résidu est cristallisé dans l'éther à 0°C. On obtient 1.82 g de composé attendu (**68%**). 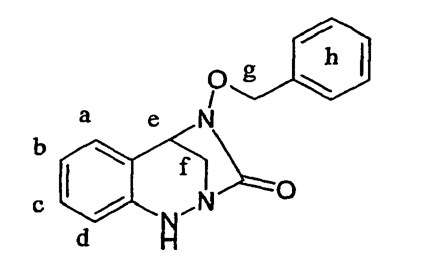
Spectre RMN : (CDCl3) : 1H : 3.16 ppm (d,J = 11) Hf1 , 1H : 3.55 ppm (dd,J = 11-2,5) Hf2 ; 1H : 3.80 ppm (d,J = 2,5) He ; 1H : 4.86 ppm , 1H : 4.98 ppm système de type AB Hg ; 1H : 6.60 ppm (dl,J = 8), 1H : 6.93 ppm (dd,J = 8-1,5) Ha et Hd ; 1H : 6.80 ppm (td,J = 8-1,5), 1H : 7.19 ppm (td,J = 8-1,5) Hb et Hc ; 1H : 7.43 ppm (m) Hh.
Spectre de Masse : 281+ M+ ; 174+ M+-(O-CH2-Ph); 131+ Ouverture du cycle carbamate ; 91+ PhCH2+.
Spectre IR : 3320 cm-1 ν(NH) ; 1746 cm-1 ν(C=O) ; 1607-1580-1490 cm-1 Aromatiques.
Spectre UV : 247 nm ε=7000 ; 290 nm ε=1800
Microanalyse :

| Calculé : | | Obtenu : | |
|---|---|---|---|
| %C : | 68.3% | %C : | 67.7% |
| %H : | 5.4% | %H : | 5.4% |
| %N : | 14.9% | %N : | 14.7% |

### Exemple 3 : [(3-oxo-2,3,4,5-tétrahydro-2,5-méthano-1H-1,2,4-benzotriazépin-4-yl)oxy]-acétate de 2-propényle

### Stade A : 4-[(carboxyméthoxy)imino]3,4-dihydro-2(1H)-cinnoléinecarboxylate de 1,1-diméthyléthyle

3 g de composé obtenu au stade C de l'exemple 1 sont solubilisés dans 25 ml de pyridine, puis, sous agitation et sous argon, on ajoute 3,9 g de carboxyméthylhydroxylamine. Au bout d'une heure, on évapore la pyridine, on reprend le résidu avec le mélange heptane-AcOEt (1:2) et NaHSO₄ (solution acqueuse à 10%). On extrait une fois l'acétate d'éthyle, et lave à l'eau. On sèche ensuite la phase organique sur MgSO₄. On filtre, évapore le solvant et 3,56 g de composé attendu (92%) sont isolés. 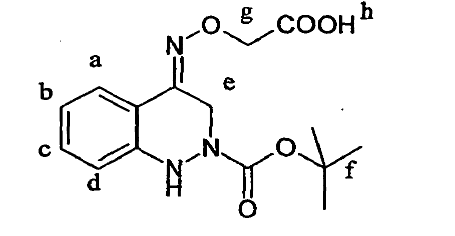
Spectre RMN : (DMSO): 9H : 1.35 ppm (s)Hf ; 2H : 4.63 ppm (s), 2H : 4.67 ppm (s) He et Hg ; 1H : 6.81 ppm (td, J = 8-1,5), 1H : 7.24 ppm (td,J = 8-1,5) Hb et Hc ; 1H : 6.96 ppm (dl,J = 8), 1H : 7.62 ppm (dl,J = 8) Ha et Hd ; 1H : 8.49 ppm (sl) NH ; 1H : 12.82 ppm (s) Hh.
Spectre de Masse : 322+ MH+ ; 344+ MNa+ ; 643+ (2M+H)+ ; 266+ MH+-tBu ; 146+ MH+-boc-(O-CH2-COOH).
Spectre IR : 3344 cm-1 ν(NH) ; 1708 cm-1 ν(C=O) ; 1638-1610-1589-1494 cm-1 ν(C=C) + aromatique.
Spectre UV : 236nm ε=14800 ; 259nm ε=12600 ; 330 nm ε=4000
Microanalyse :

| Calculé : | | Obtenu : | |
|---|---|---|---|
| %C : | 56.1% | %C : | 55.7% |
| %H : | 6% | %H : | 5.8% |
| %N : | 13.1% | %N : | 13.3% |

### Stade B : 3,4-dihydro-4-[[2-oxo-2-(2-propényloxy) éthoxy]imino]-2(1H)-cinnoléinecarboxylate de 1,1-diméthyléthyle

15,5 g de produit obtenu comme décrit au stade A sont solubilisés dans 200 ml de DMF. 12,17 g de bicarbonate de sodium et 17.53 g (12.5 ml) de bromure d'allyle sont ajoutés à la solution. Après 48 heures à température ambiante sous agitation, sous argon, on traite le milieu réactionnel avec un mélange heptane-AcOEt (1:2) et NaH₂PO₄ (solution aqueuse à 1M). Après extraction à l'heptane-AcOEt (1:2), lavage de la phase organique à l'eau puis avec une solution aqueuse saturée au bicarbonate de sodium, on sèche sur MgSO₄ et évapore le solvant. On fait cristalliser le produit dans le pentane. On isole 12.68 g de produit attendu (**81%**). 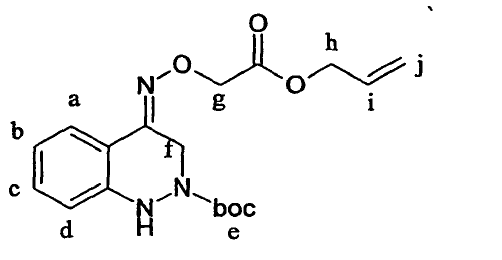
Spectre RMN : (CDCl3) 9H : 1.45 ppm (s) He ; 2H : 4.69 ppm (d) ; Hh ; 1H : 5.93 ppm (m) Hi ; 1H : 5.25 ppm (qd) Hj1 , 1H : 5.35 ppm (qd)Hj2 ; 2H : 4.76 ppm (s) Hg ; 2H : 4.80 ppm (s) Hf ; H : 6.81 ppm (d) Hd ; 1H : 6.89 ppm (td) Ha ; 1H : 7.23 ppm (td) Hb ;1H : 7.81 ppm (dd)Hc.
Spectre de Masse : 362+ MH+ ; 384+ MNa+ ; 328+ MNa+-tBu ; 369+ MNa+-tBu+CH3CN ; Présence de structure diallylé : 424+ MNa+ ; 402+ MH+ ; 346+ MH+-tBu.
Spectre IR : 3475 cm-1 ν(NH) ; 3365-3340 cm-1 ν(C=O) ; 1757-1698 cm-1 ν(C=C) ; 1645 cm-1 Aromatiques ; 1622-1608-1578 cm-1.
Spectre UV : 237 nm ε=14500 ; 259 nm ε=12000 ; 330 nm ε=3800

### Stade C : 3,4-dihydro-4-[[2-oxo-2-(2-propényloxy) éthoxy]amino]-2(1H)-cinnoléinecarboxylate de 1,1-diméthyléthyle

12,68 g de produit obtenu au stade B sont solubilisés dans 1,4 1 de méthanol. On ajoute 35,3 g de cyanoborohydrure de sodium à 0°C, puis goutte à goutte, 59,75 g d'étherate de trifluorure de bore après évaporation du méthanol, le résidu est traité par NaH₂PO₄ (solution aqueuse 1M), puis on extrait avec un mélange heptane-AcOEt (1:2). On lave la phase organique à l'eau, la sèche avec MgSO₄ et évapore le solvant. Le composé est ensuite passé sur silice (éluant heptane-tBuOMe (4:1). On isole 6.16 g de, produit attendu (**48%**).

### Stade D : dichlorhydrate de [[(1,2,3,4-tétrahydro-4-cinnoléinyl)amino]oxy]-acétate de 2-propényle

Les 6,16 g de produit obtenu au stade C sont solubilisés dans 22 ml d'acétate d'éthyle, puis on ajoute (38 ml d'une solution d'acide chlorhydrique gazeux dans l'acétate d'éthyle à 4.3M à 0°C, sous agitation et sous argon. On revient à température ambiante. Après 30 mn, le précipité est filtré, lavé à l'éther, séché sous pression réduite. On isole 5.63 g de produit attendu (**99%**).

### Stade E : [(1,5-dihydro-3-oxo-2,5-méthano-2H-1,2,4-benzotriazépin-4(3H)-yl)oxy]-acétate de 2-propényle

5,63 g de produit obtenu au stade D sont solubilisés dans 2 1 d'acétonitrile. On ajoute lentement 8,45 g de triéthylamine, puis, à 0°C, 1,658 g de diphosgène et 2,25 g de diméthylaminopyridine.On laisse remonter à température ambiante. Après une heure, On évapore l'acétonitrile et on traite avec NaH₂PO₄ (solution aqueuse 1M). On extrait à l'AcOEt et on lave la phase organique à l'eau. On sèche sur MgSO₄ et évapore le solvant. On chromatographie le résidu sur silice (éluant heptane-AcOEt (4:1)). On cristallise le composé obtenu dans l'éther à 0°C et isole 1,96 g de produit attendu (**41%**). 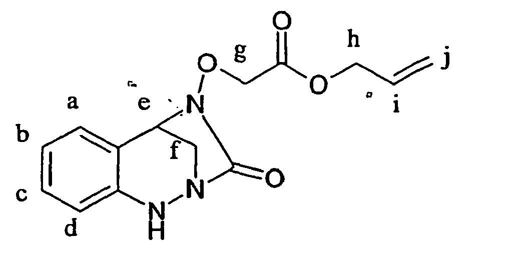
Spectre RMN : (CDCl3) 1H : 3.32 ppm (d) Hf1 ; 1H : 3.69 ppm (dd) Hf2 ; 1H : 4.83 ppm (d) He ; 2H : 4.55 ppm(s)Hg ; 2H : 4.71 ppm (d) Hh ; 1H : 5.96 ppm (m) Hi ; 1H : 5.32 ppm (qd) Hj1 ; 1H : 5.38 ppm (qd) Hj2 ; 1H : 6.64 ppm (d) Hd ; 1H : 7.28 ppm (dd) Ha ; 1H : 6.85 ppm (td) Hb ; 1H : 7.22 ppm (td) Hc.
Spectre de Masse : 290+ MH+ ; 312+ (M+Na)+ ; 601+ (2M+Na)+ ;
Spectre IR : 3320 cm-1 ν(NH) ; 1746 cm-1 ν(C=O) ; 1678-1580-1490 cm-1 Aromatiques.

### Exemple 4: [[1,5-dihydro-1-(méthylsulfonyl)-3-oxo-2,5-méthano-2H-1,2,4-benzotriazépin-4(3H)-yl]oxy]-acétate de 2-propényle

100 mg du produit obtenu à l'exemple 3 sont solubilisés dans 2 ml de CH₂Cl₂ anhydre. On ajoute ensuite à 0°C, 43,53 mg de chlorure de méthane sulfonique, puis 38,4 mg de triéthylamine, puis 46,4 mg de diméthylaminopyridine. Après 10 minutes, on évapore le solvant. Le résidu est traité par un mélange heptane-AcOEt (1:2) et NaH₂PO₄ (solution aqueuse 1M). Après extraction à l'AcOEt, puis lavage à l'eau de la phase organique et séchage sur MgSO₄, on évapore le solvant. On isole 114.5 mg de produit attendu (90%).

### Exemple 5 : sel de N-(1-méthyléthyl)-2-propanaminium de l'acide [[1,5-dihydro-1-(méthylsulfonyl)-3-oxo-2,5-méthano-2H-1,2,4-benzotriazépin-4(3H)-yl]oxy]-acétique

112 mg de produit obtenu à l'exemple 4 sont solubilisés dans 0.8 ml de THF. 35,3 mg de tétrakistriphénylphosphine palladium puis 154,2 mg de diisopropylamine sont ajoutés à la solution. La réaction est laissée 20 minutes à 0°C sous agitation et sous argon. On ajoute 0.1 ml d'éther, puis on filtre et on lave le solide avec 1 ml d'un mélange THF-éther (4:1). 99.5 mg de produit attendu (**76%**) sont isolés. 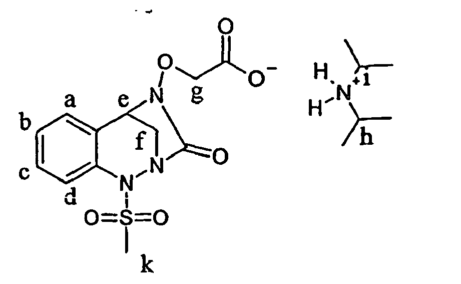
Spectre RMN : (DMSO) 12H : 1.19 ppm (d,J = 6,5) Hh ; 2H : 3.27 ppm (sept, J = 6,5) Hi ; 1H : 3.44 ppm (d,J = 9) Hf1 ; 1H : 3.65 ppm (dd, J = 2,5) Hf2 ; 1H : 5.22 ppm (d,J = 2,5) He ; 1H : 3.39 ppm (s) Hk ; 1H : 7.45 ppm (dd,J = 8-1,5) Ha ; 1H : 7.10 ppm (td,J = 8-1,5) Hb ; 1H : 7.35 ppm (td,J = 8-1,5) Hc ; 1H : 7.61 ppm (dd,J = 8-1,5) Hd.
Spectre de Masse : 102+ M+ ; 279+ Ph3P=O+ ; 326- M-
Spectre UV : 278 nm ε=1400 ; 322 nm ε=1200 ; inflexion à 260,275,286 nm.

### Exemple 6: [[1-[(benzoylamino)carbonyl]-1,5-dihydro-3-oxo-2,5-méthano-2H-1,2,4-benzotriazépin-4(3H)-yl]oxy]-acétate de 2-propényle

100 mg du produit obtenu à l'exemple 3 sont solubilisés dans 5 ml de toluène. On ajoute à 0°C 50,85 mg de isocyanate de benzoyle.On laisse remonter à température ambiante. Après 1 heure sous agitation et sous argon, on filtre et lave avec 1 ml de toluène. On sèche et isole 80 mg de produit attendu (53%).

### Exemple 7 : sel de N-(1-méthyléthyl)-2-propanaminium de l'acide [[1-(benzoylamino)carbonyl]-1,5-dihydro-3-oxo-2,5-méthano-2H-1,2,4-benzotriazépin-4-(3H)-yl]oxy]-acétique

80 mg de produit obtenu à l'exemple 6 sont solubilisés dans 0.8 ml de THF. 21,2 mg de tetrakistriphénylphosphine paladium, puis 92,6 mg de diisopropylamine sont ajoutés à la solution. La réaction est laissée 20 minutes à 0°C, sous agitation et sous argon. On ajoute 0.1 ml d'éther, puis on filtre. On lave le solide avec 1 ml d'un mélange THF-éther (4:1). 50.9 mg de produit attendu (**56%**) sont isolés. 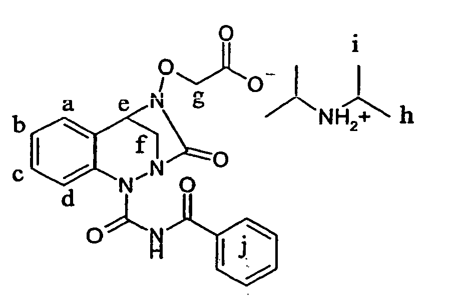
Spectre RMN : (DMSO) : 12H : 1.18 ppm (d,J = 6,5) Hh ;2H : 3.26 ppm (sept,J = 6,5) Hi ; 1H : 3.55 ppm (d,J = 11,5) Hf1 ; 1H : 3.78 ppm (dd,J = 11,5-2,5) Hf2 ; 1H : 5.30 ppm (d,J = 2,5) He ; 2H : 4.07 ppm système de type AB Hg ; 9H : 7.12-8.35 ppm (m) ; Hj ; <1H : 8.84 ppm (sl) H mobile.
Spectre de Masse : 397+ MH+ ; 395- (M-H)-
Spectre IR : Absorptions région ν(NH); 1776-1758 cm-1 ν(C=O); 1678-1630 cm-1 ν(C=O) +ν(COO) ; 1586-1501 cm-1 Amide II+Aromatiques.
Microanalyse :

| Calculé : | | Obtenu : | |
|---|---|---|---|
| %C : | 60.3% | %C : | 60% |
| %H : | 6.3% | %H : | 6.5% |
| %N : | 14.08% | %N : | 12.9% |

### Exemple 8: [[1,5-dihydro-3-oxo-1-[[(phénylsulfonyl)amino] carbonyl]-2,5-méthano-2H-1,2,4-benzotriazépin-4(3H)-yl]oxy]-acétate de 2-propényle

100 mg du produit obtenu à l'exemple 3 sont solubilisés dans 1 ml de toluène. On ajoute 63,3 mg d'isocyanate de benzène sulfonyle à 0°C puis la réaction est laissée sous agitation et sous argon à température ambiante pendant 45 mn. On purifie ce composé par chromatograhies en couches minces préparatives (éluant : heptane-AcOEt (2:1))et isole 150 mg de produit attendu (**92**%)

### Exemple 9 : Sel de bis-[N-(1-méthyléthyl)-2-propanaminium] de l'acide [[1,5-dihydro-3-oxo-1-[[(phénylsulfonyl)amino] carbonyl]-2,5-méthano-2H-1,2,4-benzotriazépin-4(3H)-yl]oxy]-acétique

148 mg de produit obtenu à l'exemple 8 sont solubilisés dans 1.5 ml de THF. 36 mg de tétrakisthiphénylphosphine palladium, puis 158,4 mg de diisopropylamine sont ajoutés à la solution. La réaction est laissée 20 minutes à 0°C, sous agitation et sous argon. On ajoute 0.1 ml d'éther, puis on filtre le précipité. On lave avec 1 ml d'un mélange THF-éther (4:1). 66 mg de produit attendu (**35%**) sont isolés. 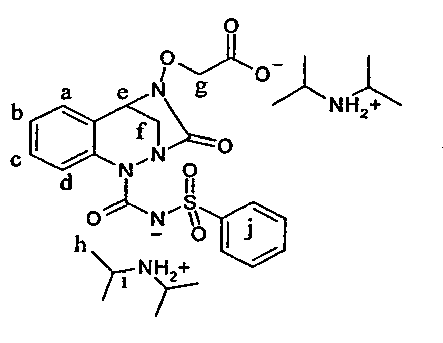
Spectre RMN : (DMSO) 24H : 1.18 ppm (d,J = 6,5) Hh ; 4H : 3.28 ppm (sept,J = 6,5) ; Hi ; 1H : 2.98 ppm (d,J = 11,5) Hf1 ; 1H : 3.44 ppm (dd, J = 11,5-2,5)Hf2 ; 1H : 4.94 ppm (d,J = 2,5) He ; 2H : 4.06 ppm ; système de type AB Hg ; 1H : 6.84 ppm (td,J = 8-1,5) Hb ; 1H : 7.13 ppm (td,J = 8-1,5) Hb et Hd ; 1H : 7.23 ppm (dd,J = 8-1,5) Ha, 1H : 8.06 ppm (dd,J = 8-1,5) Hd ; 3H : 7.37 ppm (m) Hj ; 2H : 7.78 ppm (m) Hj ; <4H : 8.49 ppm (sl) H mobiles.
Spectre de Masse : 433+ MH+ ; 431+ MH+.
Spectre IR : Absorptions région ν(NH) ; 1748 cm-1 ν(C=O)+ ν(COO-) ; 1500 cm-1 Aromatiques.

### Exemple 10 : 4,5-dihydro-α,3-dioxo-4-[2-oxo-2-(2-propényloxy)éthoxy]-2,5-méthano-2H-1,2,4-benzotriazépine-1(3H)-acétate d'éthyle

100 mg du produit obtenu à l'exemple 3 sont solubilisés dans 4 ml de CH₂Cl₂ anhydre. On ajoute ensuite 45,4 mg de triéthylamine puis, à 0°C, 61,37 mg de chloroglyoxylate d'éthyle puis 54,8 mg de diméthylaminopyridine. On laisse remonter à température ambiante. Après 15 mn, on évapore CH₂Cl₂ et le résidu est traité par un mélange heptane:AcOEt 1:1 et NaH₂PO₄ (solution aqueuse 1M). Après extraction à l'AcOEt, puis lavage da la phase organique à l'eau et sèchage sur MgSO₄, on évapore le solvante et isole 124.6 mg de produit attendu (**93%**).

### Exemple 11 : Sel de N-(1-méthyléthyl)-2-propanaminium de l'acide [(1,5-dihydro-3-oxo-2,5-méthano-2H-1,2,4-benzotriazépin-4(3H)-yl)oxy]-acétique

80,7 mg du produit obtenu à l'exemple 10 sont solubilisés dans 0.8 ml de THF. 32,2 mg de tétrakistriphénylphosphine palladium, puis 141,6 mg de diisopropylamine sont ajoutés à la solution. La réaction est laissée 20 minutes à 0°C, sous agitation et sous argon. On ajoute 0.1 ml d'éther, puis on filtre le précipité et le lave avec 1 ml d'un mélange THF-éther (4:1). 87 mg de produit attendu (**89%**) sont isolés. 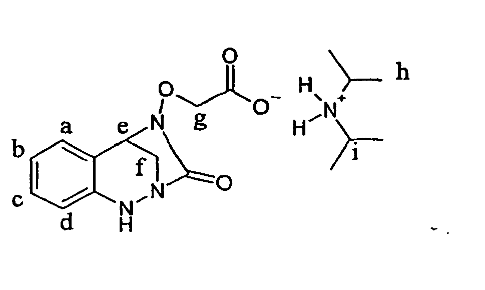
Spectre RMN : (DMSO) : 12H : 1.18 ppm (d,J = 6,5) Hh ; 2H: 3.24 pm (sept J-6,5)Hi ; 1H : 3.00 ppm (d,J = 11) Hf1 ; 1H : 3.48 ppm (dd,J = 11-2,5) Hf2 ; 1H : 4.98 ppm (d,J = 2,5) He ; 1H : 4.02 ppm (s) Hg ; 1H : 6.54 ppm (dd, J = 8-1) Ha ou Hd ; 1H : 6.65 ppm (Tt) Hd , 1H : 7.11 ppm (td,J = 8-1,5) Hb et Hc ; 1H : 7.15 ppm (dd,J = 8-1) Ha ou Hd ; 1H : 8,54 H mobile.
Spectre de Masse : 351+ MH+.
Spectre IR : Absorptions région ν(NH) ν(C=O) ; 1750 cm-1 ν(COO-)+aromatisues +déf. NH-NH2+ ; 1641-1607 -1572-1505 cm-1.
Spectre UV : 245 nm ε=7200 ; 288 nm ε=1800
Microanalyse:

| Calculé : | | Obtenu : | |
|---|---|---|---|
| %C : | 58.3% | %C : | 58.4% |
| %H : | 7.5% | %H : | 7.5% |
| %N : | 16% | %N : | 15.5% |
| | | %H2O : | 0.4% |

### Exemple 12 : 4,5-dihydro-3-oxo-4-(2-propényloxy)-2,5-méthano-2H-1,2,4-benzotriazépin-1(3H)-acétate d'éthyle

500 mg de produit obtenu à l'exemple 1 sont solubilisés dans 4 ml de DMF. On ajoute ensuite 397,2 mg de bromoacétate d'éthyle, puis, à 0°C, 114,1 mg d'hydrure de sodium (50% dans l'huile). Après 20 minutes sous agitation et sous argon, le milieu réactionnel est traité par un mélange heptane-AcOEt (1:2) et NaH₂PO₄ (solution aqueuse 1M). Après extraction à l'AcOEt, puis lavage à l'eau de la phase organique, on sèche sur MgSO₄ et évapore le solvant. Le résidu est chromatographié sur silice (éluant heptane-AcOEt 2:1). On isole 418.4 mg de produit attendu (**61%**). 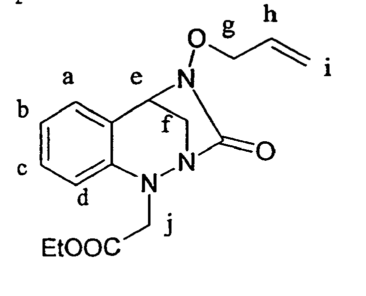
Spectre RMN : (CDCl3) : 3H : 1.27 (t,J = 7) CH3 de l'éthyle ; 2H : 4.20 ppm (q,J = 7) CH2 de l'éthyle ; 1H : 4.40 ppm, 1H : 4.47 ppm système de type AB Hj ; 1H : 3.52 ppm (d,J = 11,5)Hf1 ; 1H : 3.60 ppm (dd, J = 11,5-3) Hf2 ; 1H : 4.40 ppm (d,J = 3) He ; 2H : 4.42 ppm (masqué) Hg ; 1H : 6.01 ppm (m) Hh ; 1H : 5.31 ppm (dl,J = 10,5) Hi1, 1H : 5.35 ppm (dq,J = 17-1,5) Hi2 ; 1H : 6.46 ppm (dl,J = 8) Hd ou Ha ; 1H : 7.11 ppm (dd,J = 8-1,5) Ha ou Hd ; 1H : 6.76 ppm (tl,J = 8), 1H : 7.22 ppm (td,J = 8-1,5) Hc et Hb.
Spectre de Masse : 340+ Mna+ ; 318+ MH+ ; 260+ M+-(O=C-NH-O-CH2-CH=CH2) ; 217+ Peu ou pas de =C-NH.
Spectre IR : 1767 cm-1 ν(C=O) (Complexe) ; 1646 cm-1 ν(C=C) ; 1608-1578 cm-1 Aromatiques
Spectre UV : 250 nm ε=10000 ; 295 nm ε=2300.
Microanalyse :

| Calculé : | | Obtenu : | |
|---|---|---|---|
| %C : | 62.9% | %C : | 63% |
| %H : | 7.5% | %H : | 7.6% |
| %N : | 13.8% | %N : | 13.7% |

### Exemple 13 : 4,5-dihydro-1-méthyl-4-(2-propényloxy)-2,5-méthano-2H-1,2,4-benzotriazépin-3(1H)-one

462,5 mg du produit obtenu à l'exemple 1 sont solubilisés dans 5 ml de DMF. On ajoute ensuite 567,6 mg d'iodure de méthyle puis, à 0°C, 96 mg d'hydrure de sodium (50% dans l'huile). Après 30 minutes sous agitation et sous argon, le milieu réactionnel est traité par un mélange heptane-AcOEt (1:2) et NaH₂PO₄ (solution aqueuse 1M). Après extraction à l'AcOEt, puis lavage à l'eau de la phase organique, on sèche sur MgSO₄, et on évapore le solvant. Le résidu est chromotographié sur silice (éluant heptane:AcOEt 2:1). On isole 392 mg de produit attendu (**80%**). 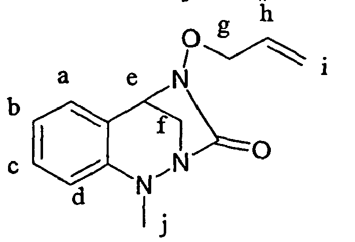
Spectre RMN : (CDCl3) : 3H : 3.29 (s) Hj ; 1H : 3.23 ppm (d,J = 11,5) Hf1 ; 1H : 3.59 ppm (dd,J = 11,5-3) Hf2 ; 1H : 4.37 ppm (d,J = 3) He ; 2H : 4.41 ppm (dl, J = 7) Hg ; 1H : 6.01 ppm (m) Hh ; 1H : 5.31 ppm (dl, J = 10,5) Hi1 ; 1H : 5.35 ppm (dq,J = 11,5-1,5) Hi2 ; 1H : 6.64 ppm (dd,J = 8-1,5), 1H : 7.07 ppm (dd,J = 8-1,5) Ha et Hd ; 1H : 6.77 ppm (td,J = 8-1,5),1H : 7.25 ppm (td,J = 8-1,5) Hc et Hb.
Spectre de Masse : 245+ M+ ; 188+ M+-(O-CH2-CH=CH2) ; 145+ M+-(NCO-O-All).
Spectre IR : 1764 cm-1 ν(C=O) ; 1644 cm-1 ν(C=C) ; 1608-1576 cm-1 Aromatiques.
Spectre UV : 253 nm ε=8900 ; 293 nm ε=2100

### Exemple 14 : 4,5-dihydro-4-(2-propényloxy)-1-(3-pyridinylméthyl)-2,5-méthano-2H-1,2,4-benzotriazépin-3(1H)-one

462,5 mg de produit obtenu à l'exemple 1 sont solubilisés dans 10 ml de DMF. On ajoute ensuite 426,5 mg de chlorhydrate de 3-chlorométhylpyridine, puis, à 0°C, 113,5 mg d'hydrure de sodium (50% dans l'huile). Après 1 heure sous agitation et sous argon, le milieu réactionnel est traité par un mélange heptane-AcOEt (1:1) et NaH₂PO₄ (solution aqueuse 1M). Après extraction à l'AcOEt, puis lavage à l'eau de la phase organique, on sèche sur MgSO₄, évapore le solvant et chromatographie le résidu sur silice (éluant heptane:AcOEt 3:1). On isole 180 mg de produit attendu (**28%**).

### Exemple 15 : 4,5-dihydro-3-oxo-N-(phénylsulfonyl)-4-(2-propényloxy)-2,5-méthano-2H-1,2,4-benzotriazépine-1(3H)-carboxamide

300 mg de produit obtenu à l'exemple 1 sont solubilisés dans 3 ml de toluène. On ajoute à 0°C 237,6 mg d'isocyanate de benzène sulfonyle. On laisse remonter à température ambiante. Après 15 mn sous agitation et sous argon. On filtre et lave les cristaux avec du toluène. On isole 480 mg de produit attendu (**89%**).

### Exemple 16 : N-benzoyl-4,5-dihydro-3-oxo-4-(2-propényloxy)-2,5-méthano-2H-1,2,4-benzotriazépine-1(3H)-carboxamide

400 mg du produit obtenu à l'exemple 1 sont solubilisés dans 5 ml de toluène. On ajoute à 0°C, 279,55 mg d'isocyanate de benzoyle. On laisse remonter à température ambiante. Après 30 mn sous agitation et sous argon, on filtre et lave les cristaux avec du toluène. On isole 429.4 mg de produit attendu (**66%**).

### Exemple 17 : 4,5-dihydro-α,3-dioxo-4-(2-propényloxy)-2,5-méthano-2H-1,2,4-benzotriazépin-1(3H)-acétate d'éthyle

400 mg du produit obtenu à l'exemple 1 sont solubilisés dans 4 ml de CH₂Cl₂ anhydre. On ajoute ensuite 192.1 mg (265 µl) de triéthylamine puis, à 0°C, 259.8 mg de chloroglyoxylate d'éthyle puis 232 mg de diméthylaminopyridine.On laisse remonter à température ambiante. Après 15 mn sous agitation et sous argon, on évapore CH₂Cl₂. Le résidu est traité par un mélange heptane:AcOEt 1:1 et NaH₂PO₄ (solution aqueuse 1M). Après extraction à l'AcOEt, puis lavage à l'eau de la phase organique et sèchage sur MgSO₄, on évapore le solvant. On isole 556 mg de produit attendu (**97%**). 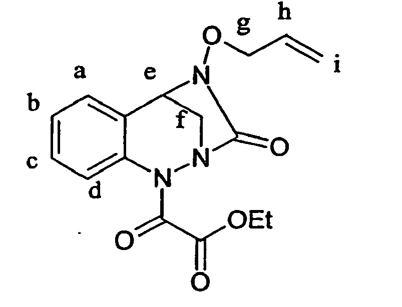
Spectre RMN : (CDCl3) : 3H : 1.40 (t,J = 7) CH3 de l'éthyle ; 2H : 4.40 ppm (q, J = 7) CH2 de l'éthyle ; 1H : 3.50 ppm (d,J = 12) Hf1 ; 1H : 3.75 ppm (dd,J = 12-3) Hf2 ; 1H : 4.40 ppm (d,J = 3) He ; 1H : 4.41 ppm (masqué) Hg ; 1H : 6.01 ppm (m) Hh ; 1H : 5.34 ppm (dl,J = 10) Hi1 ; 1H : 5.37 ppm (dq, J = 17,5-1,5) Hi2 ; 1H : 7.16 ppm (td,J = 8-1), 1H : 7.42 ppm (td,J = 8-1) Hb et Hc ; 1H : 7.23 ppm (dd,J = 8-1) Ha ; 1H : 8.40 ppm (dd,J = 8-1) Hd.
Spectre de Masse : 332+ MH+ ; 354+ MNa+ ; 395+ MNa++CH3CN ; 685+ (2M+Na)+ ; 259+ MH+-(COOEt) ; 131+ MH+-(COCOOEt)-(CO-N-OAll).
Spectre IR : 1794-1743-1699 cm-1 ν(C=O); 1602-1582 cm-1 Aromatiques ;
Spectre UV : 237 nm ε=7700 ; 260 nm ε=8800 ; inflexion à 276 nm

### Exemple 18 : 4,5-dihydro-N-méthyl-3-oxo-4-(2-propényloxy)-2,5-méthano-2H-1,2,4-benzotriazépine-1(3H)-sulfonamide

400 mg du produit obtenu à l'exemple 1 sont solubilisés dans 5 ml de CH₂Cl₂ anhydre. On ajoute ensuite 576 mg de triéthylamine à 0°C, puis 740 mg de chlorure de méthyl-sulfamoyle. Le milieu est maintenu sous agitation pendant 20 mn. On évapore CH₂Cl₂,. Le résidu est traité par un mélange heptane-AcOEt (1:1) et NaH₂PO₄ (solution aqueuse 1M). Après extraction à l'AcOEt, puis lavage à l'eau de la phase organique et sèchage sur MgSO₄, on évapore le solvant. On répète la réaction avec 1.5éq des deux réactifs ci-dessus. Le composé est ensuite chromatographié sur silice (éluant heptane-AcOEt (2:1). On isole 226 mg de produit attendu (**40%**). 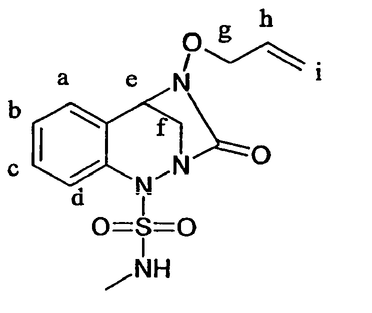
Spectre de Masse : 325+ MH+ ; 347+ MNa+ ; 388+ MNa++CH3CN ; 267+ MH+-(-O-CH2-CH=CH2) ; 232+ MH+-(-S02-NH-CH3) ; 131+ MH+-(CO-N-O-CH2-CH=CH2)-(-SO2-NH-CH3).
Spectre IR : 3380 cm-1 ν(NH) ; 1781 cm-1 ν(C=O) ; 1646 cm-1 ν(C=C) ; 1602 cm-1 Aromatiques ; 1355-1175 cm-1 ν(S02).
Spectre UV : inflexion à 226, 272, 287 nm

### Exemple 19 : 4,5-dihydro-3-oxo-N-phényl-4-(2-propényloxy)-2,5-méthano-2H-1,2,4-benzotriazépine-1(3H)-carbothioamide

40 mg de produit obtenu à l'exemple 1 sont solubilisés dans 2 ml de DMF. On ajoute ensuite 25,71 mg d'isothiocyanate de phényle à 0°C, puis 9,129 mg d'hydrue de sodium (50% dans l'huile). On laisse remonter à température ambiante. Au bout de vingt minutes, le milieu est traité par un mélange heptane-AcOEt (1:2) et NaH₂PO₄ (solution aqueuse 1M). Après extraction à l'AcOEt, puis lavage à l'eau de la phase organique et sèchage sur MgSO₄, on évapore le solvant. On triture le composé à l'éther. On isole 41.5 mg de produit attendu (**66%**). 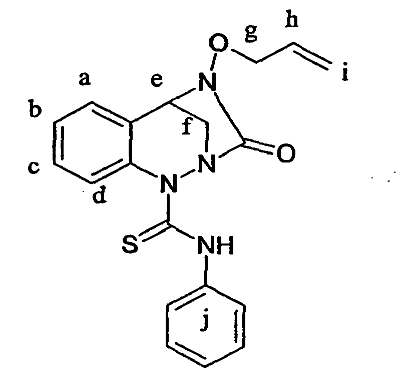
Spectre RMN : (CDCl3) : 1H : 3.68 (dd,J = 13-2) Hf1 ; 1H : 4.86 ppm (dd,J = 13-2) Hf2 ; 1H : 4.43 ppm (t,J = 2) He ; 2H : 4.22 ppm (m) Hg ; 1H : 5.91 ppm (m) Hh ; 1H : 5.21 ppm (dl,J = 10) Hi1 ; 1H : 5.27 ppm (dq, J = 17-1,5) Hi2 ; 1H : 5.52 ppm (sl) H mobile ; 1H : 7.31 ppm (td,J = 8-1), 1H : 7.50 ppm (masqué)Hb et Hc ; 1H : 7.41 ppm (dd,J = 8-1) Ha ; 1H : 9.28 ppm (dd,J = 8-1) Hd ; 5H : 7.43-7.60 ppm (m) Hj.
Spectre de Masse : 367+ MH+ ; 294+ MH+-(-N-O-CH2-CH=CH2) ; 189+ MH+-(O=C-N-O-CH2-CH=CH2)-(Ph) ; 175+ MH+-(CO-N-O-CH2-CH=CH2)-(-NH-Ph).
Spectre IR : 3468-3265 cm-1 ν(NH) ; 1745 cm-1 ν(C=O) ; 1646 cm-1 ν(C=C) ; 1605-1596-1585-1494 cm-1 Aromatiques ; 1355-1175 cm-1.
Spectre UV : 240 nm ε=17400 ; 311 nm ε=15600.

### Exemple 20 : 4,5-dihydro-1-(méthylsulfonyl)-4-(2-propényloxy)-2,5-méthano-2H-1,2,4-benzotriazépin-3(1H)-one

500 mg du produit obtenu à l'exemple 1 sont solubilisés dans 5 ml de CH₂Cl₂ anhydre. On ajoute ensuite à 0°C, 545,2 mg de chlorure de méthane sulfonyle, puis 480,8 mg de triéthylamine, puis 581 mg de diméthylaminopyridine. Après 30 mn, on évapore le CH₂Cl₂, et le résidu est traité par un mélange heptane-AcOEt (1:2) et NaH₂PO₄ (solution aqueuse 1M). Après extraction à l'AcOEt, puis lavage à l'eau de la phase organique et sèchage sur MgSO₄, on évapore le solvant. On isole 393.8 mg de produit attendu (59%). 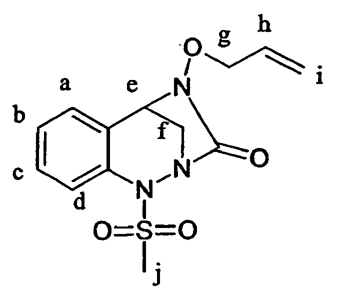
Spectre RMN : (CDCl3) : 3H : 3.41 (s) Hj ; 1H : 3.63 ppm (d) Hf1 ; 1H : 3.71 ppm (dd) Hf2 ; 1H : 4.38 ppm (d) He ; 1H : 4.43 ppm (d) Hg ; 1H : 6.01 ppm (m) Hh ; 1H : 5.35 ppm (d) Hi1 ; 1H : 5.37 ppm (dq) Hi2 ; 1H : 7.03 ppm (td) Hb ; 1H : 7.34 ppm (td) Hd ; 1H : 7.17 ppm (d)Ha ; 1H : 7.75 ppm (d) Hd.
Spectre de Masse : 309+ M+ ; 252+ M+-(-O-CH2-CH=CH2) ; 230+ M+-SO2CH3 ; 210+ 252+-(N=C=O); 174+ + M+-(-O-CH2-CH=CH2)-(SO2-CH3); 131 174+-(N=C=O) ; 103+ 131+-N2.
Spectre IR : Peu ou pas de =C-NH ; 1790 cm-1 ν(C=O) ; 1645 cm-1 ν(C=C) ; 1603-1578 cm-1 Aromatiques ; SO2 probable.
Microanalyse :

| Calculé : | | Obtenu : | |
|---|---|---|---|
| %C : | 50.5% | %C : | 62.9% |
| %H : | 4.9% | %H : | 7.5% |
| %N : | 13.6% | %N : | 13.8% |
| %S : | 10.4% | %S : | 10.4% |

### Exemple 21 : 4,5-dihydro-3-oxo-4-(2-propényloxy)-2,5-méthano-2H-1,2,4-benzotriazépine-1(3H)-carboxamide

500 mg de produit obtenu à l'exemple 1 sont solubilisés dans 35 ml de CH₂Cl₂. On ajoute ensuite 642 mg de triéthylamine puis, à 0°C, 982,63 mg de diphosgène et enfin 290 mg de diméthylamino pyridine. On laisse remonter à températutre ambiante. Après 20 minutes sous agitation, sous argon, on ajoute quelques gouttes de CH₂Cl₂ saturé en ammoniaque, puis évapore le solvant puis traite le résidu avec un mélange heptane-AcOEt (1:2) et NaH₂PO₄ (solution aqueuse 1M). Après extraction à l'AcOEt, puis lavage à l'eau de la phase organique et sèchage sur MgSO₄, on évapore le solvant. Le produit repris à l'éther, cristallise. On isole 286 mg de produit attendu (**48%**). 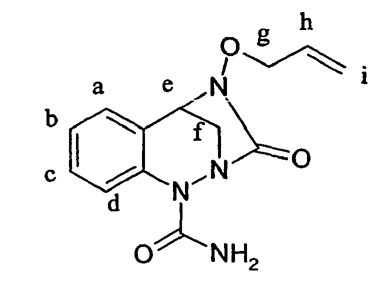
Spectre RMN : (CDCl3) : 1H : 3.36 ppm (d,J = 11,5) Hf1 ; 1H : 3.73 ppm (dd,J = 11,5-3) Hf2 ; 1H : 4.40 ppm (d,J = 3)He ; 2H : 4.44 ppm (dl,J = 6,5) Hg ; 2H : 6.02 ppm (m) Hh ; 1H : 5.35 ppm (dl, J = 10) Hi1 ; 1H : 5.37 ppm (dq,J = 17-1,5) Hi2 ; 1H : 7.01 ppm (td,J = 8-1), 1H : 7.35 ppm (td,J = 8-1)Hb et Hc ; 1H : 7.15 ppm (dd,J = 8-1) Ha ; 1H : 8.40 ppm (dd,J = 8-1)Hd ; 1H : 6.52 ppm (St) H mobile ; 1H : 4.96 ppm (sl) et 6, 96 (sl)NH₂ mobile.
Spectre UV : 241 nm ε=10000 ; inflexion à 277.3 nm.
Spectre IR : 3475 cm-1 ν(NH); 1774-1700 cm-1 ν(C=O) ; 1569 cm-1 Aromatiques.
Microanalyse :

| Calculé : Obtenu : | | | |
|---|---|---|---|
| %C : | 56.9% | %C : | 56.6% |
| %H : | 5.1% | %H : | 5.1% |
| %N : | 20.4% | %N : | 20.4% |

### Exemple 22 : 4,5-dihydro-3-oxo-N-(phénylméthyl)-4-(2-propényloxy)-2,5-méthano-2H-1,2,4-benzotriazépine-1(3H)-carboxamide

500 mg de produit obtenu à l'exemple 1 sont solubilisés dans 220 ml de CH₂Cl₂ anhydre. On ajoute ensuite à 0°C, 428 mg de thriéthylamine, puis 436,7 mg de diphosgène, puis 290 mg de diméthylamino pyridine. 20 minutes après, 254 mg de benzylamine sont ajoutés . On laisse remonter à température ambiante. On évapore le CH₂Cl₂, le résidu est traité par un mélange heptane-AcOEt (1:2) et NaH₂PO₄ (solution aqueuse 1M). Après extraction à l'AcOEt, puis lavage à l'eau de la phase organique, on sèche sur MgSO₄, et évapore le solvant. On isole 132 mg de produit attendu (**17%**). 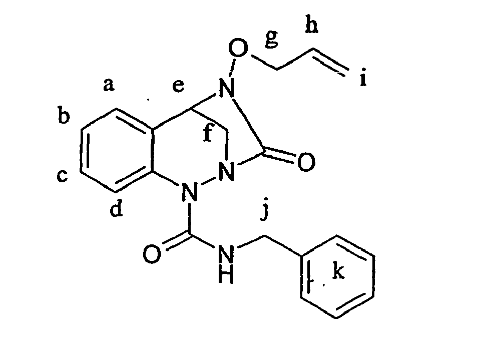
Spectre RMN : (CDCl3) : 1H : 3.31 ppm (d,J = 11,5) Hf1 ; 1H : 3.68 ppm (dd,J = 11,5-3) Hf2 ; 1H : 4.39 ppm (d,J = 3) He ; 2H : 4.43 ppm (dd,J = 6) Hg ; 1H : 6.01 ppm (m) Hh ; 1H : 5.33 ppm (d,J = 10) Hi1 ; 1H : 5.36 ppm (dq,J = 17-1,5) Hi2 ; 1H : 2H : 4.51 ppm (m) Hj ; 1H : 7.08 ppm (tl,J = 5,5) NH mobile ; 1H : 6.99 ppm (td, J = 8,1) Hb ; 1H : 7.14 ppm (dd, J = 8-1) Ha ; 6H : 7.27-7.40 ppm (m) Hk + Hc ; 1H : 8.45 ppm (dl,J = 8) Hd.
Spectre de Masse : 365+ MH+ ; 387+ Mna+ ; 428+ MNa+-CH3CN ; 751+ (2M+Na)+ ; 322+ MH+-(-O-CH2-CH=CH2) ; 292+ MH+-(-N-O-CH2-CH=CH2) ; 265+ MH+-(CO-N-O-CH2-CH=CH2).
Spectre IR : 3428 cm-1 ν(NH) ; 1783-1689 cm-1 ν(C=O) ; 1645 cm-1 ν(C=C) ; 1605-1585-1575-1505 cm-1 Aromatiques.
Spectre UV : max 244 nm ε=12800 ; infl 279, 288 nm.

### Exemple 23 : 4,5-dihydro-1-(phénylméthyl)-4-(2-propényloxy)-2,5-méthano-2H-1,2,4-benzotriazépin-3(1H)-one

300 mg de produit obtenu à l'exemple 1 sont solubilisés dans 3 ml de DMF. On ajoute à 0°C 180,6 mg de chlorure de benzyle puis, 68,5 mg d'hydrure de sodium (50% dans l'huile). Après 5 minutes sous agitation, sous argon, à 0°C, on rajoute 3 ml de DMF. Après 20 minutes, à 0°C, on rajoute du chlorure de benzyle et de l'hydrure de sodium (mêmes quantités). Après 10 minutes, le milieu réactionnel est traité par un mélange heptane-AcOEt (1:2) et NaH₂PO₄ (solution aqueuse 1M). Après extraction à l'AcOEt, puis lavage à l'eau de la phase organique et séchage sur MgSO₄, on évapore le solvant. Le produit est cristallisé dans l'éther. On isole 95.8 mg de produit attendu (23%).

### Exemple 24 : 4,5-dihydro-3-oxo-4-(2-propényloxy)-2,5-méthano-2H-1,2,4-benzotriazépin-1(3H)-acétate de 1,1-diméthyléthyle

1,2 g de produit obtenu à l'exemple 1 sont solubilisés dans 15 ml de DMF anhydre. On ajoute ensuite à 0°C, 1,21 g de bromoacétate de terbutyle, puis 271 mg d'hydrure de sodium (50% dans l'huile). On laisse à 0°C pendant 15 minutes. Le milieu réactionnel est traité par un mélange heptane-AcOEt (1:2) et NaH₂PO₄ (solution aqueuse 1M). Après extraction à l'AcOEt, puis lavage à l'eau de la phase organique et sèchage sur MgSO₄, on évapore le solvant. On chromatographie le résidu sur silice (éluant : heptane-AcOEt 1:2), et isole 1.52 g d'ester attendu (85%).

### Exemple 25 : Acide 4,5-dihydro-3-oxo-4-(2-propényloxy)-2,5-méthano-2H-1,2,4-benzotriazépin-1(3H)-acétique

On solubilise l'ester obtenu à l'exemple 24 dans 2.5 cm³ de CH2Cl2 et 7.5 cm³ d'acide trifluoroacétique. Au bout de 15 mn on évapore le solvant par entraînement au toluène, puis on fait cristalliser le composé dans l'éther. On obtient 519 mg d'acide attendu (**41%**).

### Exemple 26 : 4,5-dihydro-3-oxo-4-(2-propényloxy)-N-propyl-2,5-méthano-2H-1,2,4-benzotriazépin-1(3H)-acétamide

On solubilise 480 mg d'acide obtenu à l'exemple 25 dans 5 ml de DMF. 336,5 mg d'hydrate de 1-hydroxy benzetriazole, puis 350 mg de chlorhydrate de 1-(3-diméthylamino propyl)-3-éthylcarbodiimide sont ajoutés à 0°C. Au bout de 20 mn à 0°C, on additionne 107,9 g de propylamine, puis on laisse 20 mn à 0°C. Le milieu réactionnel est traité par un mélange heptane-AcOEt (1:2) et NaH₂PO₄ (solution aqueuse 1M). Après extraction à l'AcOEt, puis lavage à l'eau de la phase organique, on sèche sur MgSO₄ et évapore le solvant. On chromatographie le résidu sur silice (éluant CH₂Cl₂ ; 6% Acétone). On isole 207 mg de produit attendu (**38%**).

### Exemple 27 : Sel de sodium de 4,5-dihydro-3-oxo-4-(sulfooxy)-2,5-méthano-2H-1,2,4-benzotriazépin-1(3H)-acétate d'éthyle

410 mg du produit obtenu à l'exemple 12 sont solubilisés dans 4 ml de CH₂Cl₂. 155.6 mg d'acide acétique puis 746 mg de tetrakistriphénylphosphène palladium sont ajoutés à la solution. Après 30 minutes sous agitation et sous argon, on évapore le solvant et le résidu est chromatographié sur silice (éluant CH₂Cl₂ ; Acétone-CH₂Cl₂ ; Acétone CH₂Cl₂ +0,1% NEt₃ (100 ml)). Après évaporation des fractions, on ajoute au résidu 4 cm3 de pyridine puis 764 mg de complexe SO₃-pyridine et on laisse sous agitation et sous argon 2 heures. On obtient le sel de 1-propényltriphénylphosphonium de 4,5-dihydro-3-oxo-4-(sulfooxy)-2,5-méthano-2*H*-1,2,4-benzotriazépin-1(3*H*)-acétate d'éthyle en solution dans le milieu réactionnel. Le produit est ensuite passé sur résine DOWEX 50*8 sous forme Na+ en éluant par H₂O :10% THF. On évapore le THF, on lyophilise les fractions correspondantes, et pour finir on reprend à l'acétone pour éliminer Na₂SO₄ formé. On isole 182 mg de produit attendu (**37%**).

### Exemple 28 : Sel de sodium de 4,5-dihydro-1-méthyl-4-(sulfooxy)-2,5-méthano-2H-1,2,4-benzotriazépin-3(1H)-one

392 mg de produit obtenu à l'exemple 13 sont solubilisés dans 4 cm3 de CH₂Cl₂ . 192 mg d'acide acétique puis 924,47 mg de tétrakistriphénylphosphène palladium sont ajoutés à la solution. Après 30 mn sous agitation et sous argon, on ajoute 4 cm3 de pyridine puis 764 mg de complexe SO₃-pyridine et on laisse sous agitation et sous argon 2 heures. Le solvant est évaporé. On isole le sel de triphénylphosphosium attendu par chromatographie sur plaque de silice (20% acétone + 0,1% triéthylamine). On isole la silice contenant le produit attendu, et extrait ce dernier avec 25 ml de CH₂Cl₂-15% MeOH. Le produit est ensuite passé sur résine DOWEX 50*8 sous forme Na+ en éluant par H₂O:10% THF. On évapore le THF, on lyophilise les fractions correspondantes, et pour finir on reprend à l'acétone pour éliminer Na₂SO₄ formé. On isole 220 mg de produit attendu (**45%**).
Spectre RMN :(DMSO) : 3H : 3.16 ppm (s) Hg ; 1H : 3.19 ppm (d,J = 11,5) Hf1 ; 1H : 3.54 ppm (dd,J = 11,5-3) Hf2 ; 1H : 4.73 ppm (d,J = 3) He ; 1H : 6.74 ppm (dl,J = 8), 1H : 7.05 ppm (dd,J = 8-1) Ha et Hd ; 1H : 6.76 ppm (td,J = 8-1), 1H : 7.24 ppm (td,J = 8-1) Hc et Hb
Spectre de Masse : 279+ Ph3P=O+ ; 284+ MH+
Spectre IR : 3475 cm-1 : aromatiques.
Spectre UV : (EtOH-HCl) : 242 nm ε=7300 ; 296 nm ε=1700.

### Exemple 29 : Sel de sodium de 4,5,-dihydro-1-(3-pyridinylméthyl)-4-(sulfooxy)-2,5-méthano-2H-1,2,4-benzotriazépin-3(1H)-one

170 mg de produit obtenu à l'exemple 14 sont solubilisés dans 2 ml de CH₂Cl₂. 63,3 mg d'acide acétique puis 304,7 mg de tétrakistriphénylphosphène palladium sont ajoutés à la solution. Après 30 mn sous agitation et sous argon, on ajoute 2 cm3 de pyridine directement dans le CH₂Cl₂, puis 246 mg de complexe SO3-pyridine et laisse sous agitation et sous argon pendant 2 heures. On évapore le solvant et chromotographie le résidu sur plaque de silice (20% acétone + 0,1% triéthylamine). On isole la silice contenant le produit attendu et extrait celui-ci avec 25 cm3 de CH₂Cl₂-15% MeOH. Le produit est ensuite passé sur résine DOWEX 50W*8 sous forme Na+ en éluant par H₂O:10% THF. On évapore le THF, lyophilise les fractions correspondantes, et pour finir on reprend a l'acétone pour éliminer Na₂SO₄ formé. On isole 54 mg de produit attendu (**27%**).
Spectre RMN :(DMSO) :1H : 3.15 ppm (d,J = 11,5) Hf1 ; 1H : 3.52 ,ppm (dd,J = 11,5-2,5) Hf2 ; 1H : 4.76 ppm (d,J = 2,5) He ; 1H : 4.64 ppm (d,J AB = 16) Hg1 ; 1H : 4.89 ppm (d,J AB = 16)Hg2 ; 1H : 6.85 ppm (dl,J = 8), 1H : 7.09 ppm (dd,J = 8-1)Ha et Hd ; 1H : 6.78 ppm (td,J = 8-1), 1H : 7.23 ppm (td,J = 8-1) Hb et Hc ; 7.38-8.63 ppm (m)Hh
Spectre de Masse : 363+ (M'+2H)+ ; 385⁺(M'+H+Na) ; 747+(2M'+2H+Na)+ ; 361+ M+ ; 723+ (2M'+H) ; 745+(2M'+Na)
Spectre IR : Absorption dans la région v(NH); 1762 cm-1 v(C=O) ; 1604-1575 cm-1 Hétérocycle + Aromatiques.

### ETUDE PHARMACOLOGIQUE DES PRODUITS DE L'INVENTION

### I/ Activité antibactérienne in vitro, méthode des dilutions en milieu liquide

On prépare une série de tubes dans lesquels on répartit la même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits des exemples 11, 14 et 28.

Ces composés ont les activités regroupées dans le tableau suivant :

| **Gram-positif CMI µg/ml à 24 heures** | |
|---|---|
| S. aureus SG511 | 80 - 160 |
| S. pyogenes A561 | 40 - 160 |

| **Gram-négatif CMI µg/ml à 24 heures** | |
|---|---|
| E. coli UC1894 | 20 - 80 |
| E. coli 1507E | 20 - 160 |
| E. coli DC2 | 20 - 80 |
| E. cloacae 1321E | 40 - 80 |

Les composés selon l'invention montrent donc une activité anti-bactérienne.

### II/ ACTIVITE INHIBITRICE DE β-LACTAMASES

Les composés de formule (I) et leurs sels pharmaceutiquement acceptables présentent des activités inhibitrices marquées contre les β-lactamases de diverses souches bactériennes et ces propriétés thérapeutiquement intéressantes peuvent être déterminées in vitro sur des β-lactamases isolées :

### A. Préparation des β-lactamases Tem-1 et P99

Les β-lactamases sont isolées à partir de souches bactériennes résistantes aux pénicillines et aux céphalosporines (Tem1 et P99 sont respectivement produites par *E. coli* 250HT21 et *E. Cloacae* 293HT6). Les bactéries sont cultivées dans du bouillon coeur-cervelle à 37g/l(DIFCO), à 37°C. Elles sont récoltées en fin de phase exponentielle, refroidies et centrifugées. Les culots bactériens sont repris dans du tampon Phosphate de sodium 50mM, pH 7.0 et à nouveau centrifugés. Les bactéries sont reprises dans deux volumes de ce même tampon et lysées au moyen d'une French-Press maintenue à 4°C. Après une centrifugation 1h à 100 000G, à 4°C, les surnageants contenant la fraction soluble des extraits bactériens sont récupérés et congelés à -80°C.

### B. Détermination de l'activité β-lactamases

La méthode utilise comme substrat la Nitrocéfine (OXOID), céphalosporine chromogène, dont le produit d'hydrolyse par les B-lactamases est rouge et absorbe à 485nm. L'activité β-lactamase est déterminée en cinétique par la mesure de la variation d'absorbance à 485nm résultant de l'hydrolyse du substrat sur un spectrophotomètre de plaques (Spectra Max Plus de Molecular Devices). Les expériences se font à 37°C. La quantité d'enzyme a été normalisée et les mesures se font en vitesse initiale.

### C. Détermination de l'activité inhibitrice des β-lactamases

Deux mesures sont effectuées, sans préincubation et avec préincubation de l'enzyme et de l'inhibiteur (5mn), afin de tester l' irréversibilité de la réaction. Les produits sont testés à 6 ou 8 concentrations en duplicate. Le mélange réactionnel contient 100µM de Nitrocéfine et du tampon phosphate de sodium 50mM pH7.0.

### D. Calculs des CI50

Les Vitesses d'hydrolyse sont mesurées avec et sans inhibiteur. On détermine la concentration d'inhibiteur qui inhibe de 50% la réaction d'hydrolyse de la Nitrocéfine par l'enzyme (CI50). Le traitement des données est réalisé à l'aide du logiciel GraFit (Erathycus Software).

| **EXEMPLE n°** | **CI₅₀ nM/TEM1** | **CI₅₀ nM/P99** |
|---|---|---|
| 5 | 5,7 x 10⁻⁴ M | 4,6 x 10⁻⁴ M |
| 7 | 1,1 x 10⁻⁴ M | 6,3 x 10⁻⁵ M |
| 9 | 1,6 x 10⁻⁴ M | 1,8 x 10⁻⁴ M |
| 11 | 1,4 x 10⁻⁵ M | 1,5 x 10⁻⁵ M |
| 14 | 2,6 x 10⁻⁵ M | 1,7 x 10⁻⁵ M |
| 15 | 4,5 x 10⁻⁴ M | 1,1 x 10⁻⁴ M |
| 27 | 7,5 x 10⁻⁶ M | 5,3 x 10⁻⁷ M |
| 28 | 1,2 x 10⁻⁵ M | 3,7 x 10⁻⁵ M |

| | | |
|---|---|---|
| CI₅₀ après 5 mn de pré incubation avec l'enzyme. | | |

### Exemples de composition pharmaceutique :

1) On a préparé une composition pharmaceutique pour injection dont les ingrédients sont les suivants :
   - composé de l'exemple 11 500 mg
   - excipient aqueux stérile q.s.p. 10 ml
2) On a préparé une composition pharmaceutique (lyophilisat) pour injection renfermant :
   - d'une part : composé de l'exemple 9 500 mg
   - d'autre part : Céfotaxime 1 g
   - excipient aqueux stérile q.s.p 5 ml
Les deux principes actifs peuvent, si désiré, être introduits séparément dans deux ampoules ou flacons distincts.

## Revendications

1. Un composé répondant à la formule (I)ou l'un de ses sels avec les bases ou les acides : 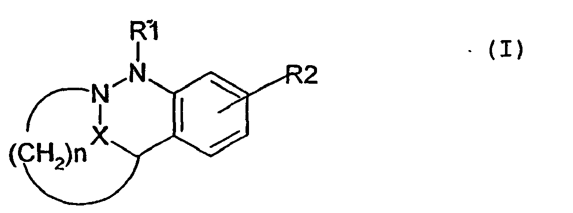 dans laquelle :
n est égal à 1 ou 2,
R₁ représente un atome d'hydrogène, un radical alkyle renfermant jusqu'à 8 atomes de carbone, un radical (CH₂)_{n'}R^{o}₁ dans lequel n' est égal à O ou 1 et R^{o}₁ représente un radical aryle renfermant jusqu'à 12 atomes de carbone, hétéroaryle renfermant jusqu'à 15 atomes de carbone et un ou plusieurs hétéroatomes choisis parmi les atomes d'azote, de soufre et d'oxygène, COR', CONR'R", CSNR'R", COCOOR', SO₂NR'R", SO₂R', CO₂R' ou CN,
R' représentant un atome d'hydrogène, un radical alkyle, alkényle renfermant jusqu'à 8 atomes de carbone, aralkyle renfermant jusqu'à 12 atomes de carbone ou aryle renfermant jusqu'à 12 atomes de carbone,
R" représentant un atome d'hydrogène, un radical alkyle renfermant jusqu'à 8 atomes de carbone, aryl renfermant jusqu'à 12 atomes de carbone, aralkyle renfermant jusqu'à 12 atomes de carbone, SO₂-R' ou COR', R' étant défini comme ci-dessus,
R₂ représente un atome d'hydrogène ou un ou plusieurs substituants choisis dans le groupe constitué par les radicaux halogeno, alkyl, OH, Oalkyl, NO₂, NH₂, NHalkyl, N(alkyl)₂, NHCOalkyl, NHSO₂alkyl, CONHalkyl, SO₂NHalkyl, COOH, COOalkyl, CN, OSO₂alkyl, NHCONHalkyl et COalkyl, alkyl renfermant jusqu'à 8 atomes de carbone,
X représente un groupement divalent -C(O)-N(OR₃)- relié à l'atome d'azote par l'atome de carbone, dans lequel R₃ est choisi dans le groupe constitué par un atome d'hydrogène et les radicaux R, Y, Y₁, Y₂, et Y₃,
R est choisi dans le groupe constitué par un radical alkyle renfermant jusqu'à 6 atomes de carbone, éventuellement substitué par un radical pyridyle ou carbamoyle, un radical alkényle renfermant jusqu'à 8 atomes de carbone, aryle renfermant jusqu'à 12 atomes de carbone ou aralkyle renfermant jusqu'à 12 atomes de carbone, le noyau du radical aryle ou aralkyle étant éventuellement substitué par un radical OH, NH₂, NO₂, alkyle renfermant de jusqu'à 8 atomes de carbone, alkoxy renfermant jusqu'à 8 atomes de carbone ou par un ou plusieurs atomes d'halogène,
Y est choisi dans le groupe constitué par les radicaux COR, COOH, COOR, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂,
Y₁ est choisi dans le groupe constitué par les radicaux SO₂R, SO₂NHCOR, SO₂NHCOOR, SO₂NHCONHR et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NR tétrazole, NR tétrazole substitué par le radical R et NRSO₂R, R étant défini comme ci-dessus.

2. Composé selon la revendication 1, **caractérisé en ce que** n est égal à 1.

3. Composé selon la revendication 1 ou la revendication 2, **caractérisé en ce que** R₂ représente un atome d'hydrogène.

4. Composé selon l'une des revendications 1 à 3, **caractérisé en ce que** R₁ représente un atome d'hydrogène, un radical alkyle renfermant jusqu'à 8 atomes de carbone, un radical (CH₂)_{n'}R^{o}₁ dans lequel n' est égal à O ou 1 et R^{o}₁ représente un radical aryle renfermant jusqu'à 12 atomes de carbone, hétéroaryle renfermant jusqu'à 15 atomes de carbone et un ou plusieurs hétéroatomes choisis parmi les atomes d'azote, de soufre et d'oxygène, CONR'R", CSNR'R", COCOOR', SO₂NR'R", SO₂R' ou CO₂R', R' et R" étant définis comme à la revendication 1.

5. Composé selon l'une des revendications 1 à 4 **caractérisé en ce que** X représente un groupement divalent -C(O)-N(OR₃)- dans lequel R₃ est choisi dans le groupe constitué par un atome d'hydrogène et les radicaux R, Y, Y₁, R, Y et Y₁ étant définis comme à la revendication 1.

6. Composés de formule (I), telle que définie à la revendication 1, dont les noms suivent :
- l'acide [[1,5-dihydro-1-(méthylsulfonyl)-3-oxo-2,5-méthano-2*H*-1,2,4-benzotriazépin-4(3*H*)-yl]oxy]-acétique,
- l'acide[[1-[(benzoylamino)carbonyl]-1,5-dihydro-3-oxo-2,5-méthano-2*H*-1,2,4-benzotriazépin-4(3*H*)-yl]oxy]-acétique,
- l'acide[[1,5-dihydro-3-oxo-1-[(phénylsulfonyl)amino carbonyl]-2,5-méthano-2*H*-1,2,4-benzotriazépin-4(3*H*)-yl]oxy]-acétique,
- l'acide [(1,5-dihydro-3-oxo-2,5-méthano-2*H*-1,2,4-benzotriazépin-4(3*H*)-yl)oxy]-acétique,
- la 4,5-dihydro-1-méthyl-4-(sulfooxy)-2,5-méthano-2*H-*1,2,4-benzotriazépin-3(1*H*)-one,
- la 4,5-dihydro-4-(2-propényloxy)-1-(3-pyridinylméthyl)-2,5-méthano-2*H*-1,2,4-benzotriazépin-3(1*H*)-one,
- le 4,5-dihydro-3-oxo-*N*-(phénylsulfonyl)-4-(2-propényloxy)-2,5-méthano-2*H*-1,2,4-benzotriazépine-1(3*H*)-carboxamide,
- le *N*-benzoyl-4,5-dihydro-3-oxo-4-(2-propényloxy)-2,5-méthano-2*H*-1,2,4-benzotriazépine-1(3*H*)-carboxamide,
- le 4,5-dihydro-α,3-dioxo-4-(2-propényloxy)-2,5-méthano-2*H*-1,2,4-benzotriazépin-1(3*H*)-acétate d'éthyle,
- le 4,5-dihydro-3-oxo-4-(sulfooxy)-2,5-méthano-2*H-*1,2,4-benzotriazépin-1(3*H*)-acétate d'éthyle,
ainsi que leurs sels tels que définis à la revendication 1.

7. Procédé de préparation d'un composé selon l'une des revendications 1 à 6, **caractérisé en ce qu'**il comporte :
a) une étape au cours de laquelle on fait réagir avec un agent de carbonylation, le cas échéant en présence d'une base, un composé de formule (II) : 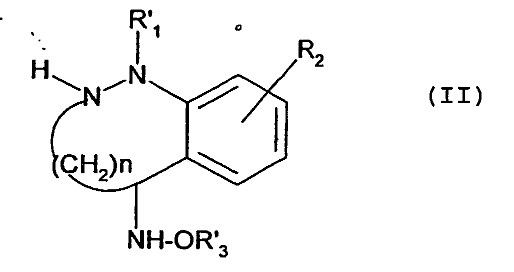 dans laquelle :
R'₁ représente un radical R₁ ou un radical qui est un précurseur de R₁, R₂ et n sont tels que définis à la revendication 1 et R'₃ représente un groupement protecteur de l'hydroxy ou un radical Rp, Yp, Y₁p, Y₂p ou Y₃p, ces radicaux ayant respectivement les valeurs de R, Y, Y₁, Y₂ et Y₃ indiquées à la revendication 1, dans lesquelles les éventuelles fonctions réactives présentes sont le cas échéant protégées, en vue d'obtenir un composé intermédiaire de formule (III) : 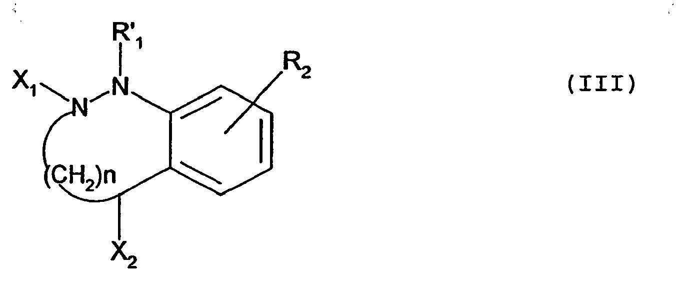
dans laquelle :
R'₁, R₂ et n ont les mêmes significations que ci-dessus et soit X₁ est un atome d'hydrogène et X₂ représente un groupement -N(OR'₃)-CO-X₃, R'₃ étant tel que défini précédemment et X₃ représentant le reste de l'agent de carbonylation, soit X₂ représente un groupement -NH-OR'₃ et X₁ représente un groupement CO-X₃, X₃ étant défini comme précédemment ;
b) une étape au cours de laquelle on cyclise l'intermédiaire obtenu précédemment, en présence d'une base ; et **en ce que** :
c) le cas échéant, l'étape a) est précédée et/ou l'étape b) est suivie de l'une ou de plusieurs des réactions suivantes, dans un ordre approprié :
- protection des fonctions réactives,
- déprotection des fonctions réactives,
- estérification
- saponification,
- sulfatation,
- phosphatation
- amidification,
- acylation,
- sulfonylation ; .
- alkylation ;
- formation d'un groupe urée ;
- introduction d'un groupement tétrazole;
- réduction d'acides carboxyliques ;
- déshydratation d'amide en nitrile ;
- salification ;
- échange d'ions ;
- séparation de d'énantiomères ;
- nitration ;
- réduction d'un nitro en amino ;
- halogénation ;
- carbamoylation ;
- cyanuration.

8. Procédé selon la revendication 7, **caractérisé en ce que** l'agent de carbonylation est choisi dans le groupe constitué par le phosgène, le diphosgène, le triphosgène, les chloroformiates d'aryle, d'aralkyle, d'alkyle et d'alkényle, les dicarbonates d'alkyle, le carbonyl-diimidazole et leurs mélanges.

9. Procédé selon la revendication 7 ou la revendication 8, **caractérisé en ce que** la réaction de carbonylation a lieu en présence d'une base.

10. Procédé selon l'une des revendications 7 à 9, **caractérisé en ce que** dans l'étape b) la base est choisie dans le groupe constitué par les amines, les hydrures, alcoolates, amidures ou carbonates de métaux alcalins ou alcalino-terreux.

11. Procédé selon la revendication 10, **caractérisé en ce que** la base est une amine.

12. Procédé selon l'une quelconque des revendications 7 à 11, **caractérisé en ce que** le composé de formule (II) est obtenu par un procédé selon lequel on traite un composé de formule (IV) : 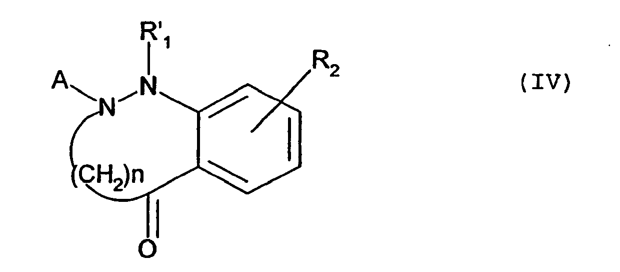 dans laquelle R'₁, R₂ et n sont définis comme à la revendication 7, et A représente un atome d'hydrogène ou un groupement protecteur de l'azote, par un agent de réduction, pour obtenir un composé de formule (V) : 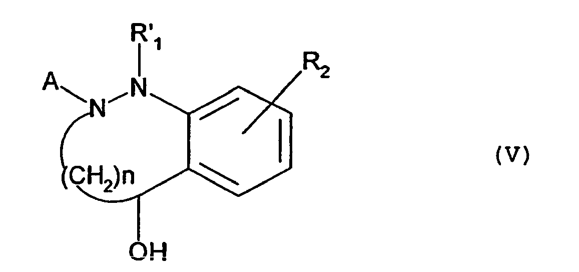 dans laquelle A, R'₁, R₂ et n conservent leur signification précitée, dans lequel, le cas échéant, l'on remplace le groupement OH par un groupe partant, pour obtenir un composé de formule (VI) : 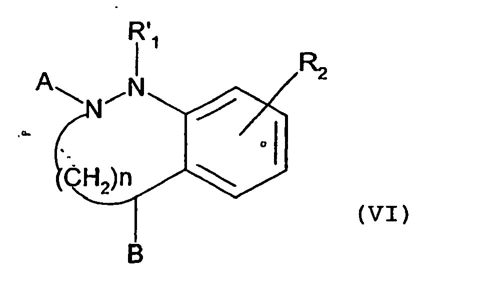 dans laquelle A, R'₁, R₂, et n conservent leur signification précitée et B représente un groupe partant, que l'on traite par un composé de formule NH₂-OR'₃, R'₃ conservant la signification indiquée à la revendication 7, puis, le cas échéant, par un agent de déprotection approprié de l'atome d'azote.

13. Procédé selon l'une quelconque des revendications 7 à 11, **caractérisé en ce que** le composé de formule (II) est obtenu par un procédé selon lequel on traite un composé de formule (IV) telle que définie à la revendication 12, par un composé de formule H₂N-OR'₃, pour obtenir un composé de formule (VII) : 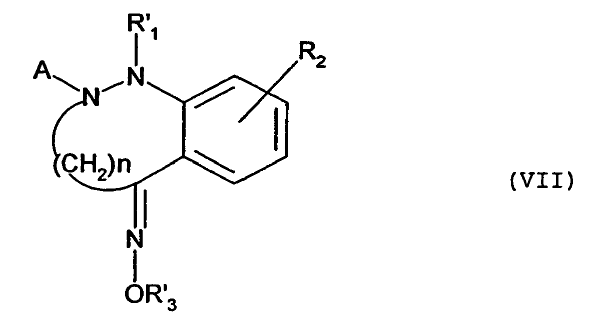 dans laquelle A est défini comme à la revendication 12 et R'₁, R₂, n et R'₃ sont définis comme à la revendication 7, que l'on fait réagir avec un agent de réduction pour obtenir un composé de formule (VIII) : 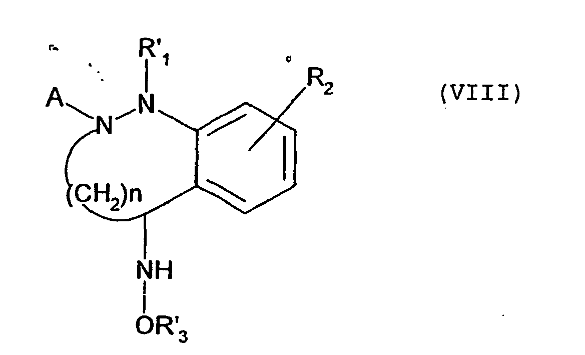 dans laquelle A, R'₁, R₂, n et R'₃ sont définis comme précédemment, que l'on traite, le cas échéant, par un agent de déprotection de l'atome d'azote approprié.

14. A titre de médicaments, les produits tels que définis à l'une quelconque des revendications 1 à 5 ainsi que leurs sels avec les acides et les bases pharmaceutiquement acceptables.

15. A titre de médicaments, les produits tels que définis à la revendication 6, ainsi que leurs sels avec les acides et les bases pharmaceutiquement acceptables.

16. Compositions pharmaceutiques contenant, à titre de principe actif, au moins un médicament selon l'une des revendications 14 ou 15.

17. Composé de formule générale (III) ou l'un de ses sels avec un acide, notamment son chlorhydrate et son trifluoroacétate : 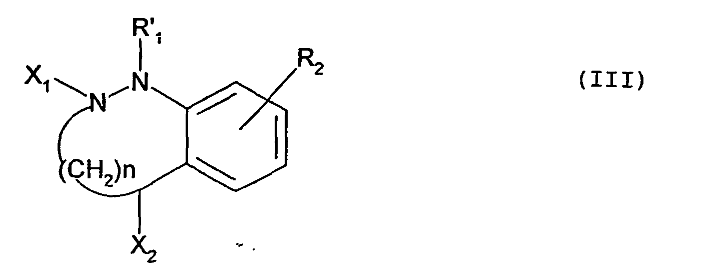 dans laquelle :
R'₁, R₂, X₁, X₂ et n ont les mêmes significations qu'à la revendication 7.

18. Composé de formule générale (II) ou l'un de ses sels avec acide, notamment son chlorhydrate et son trifluoroacétate : 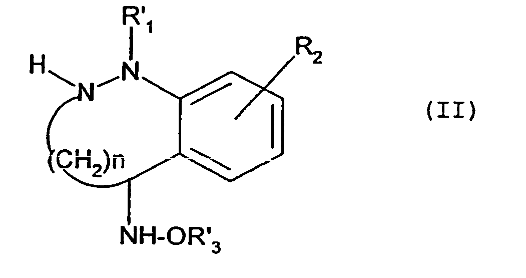 dans laquelle R'₁, R₂, R'₃ et n ont les mêmes significations qu'à la revendication 7.

19. Composés de formule (IV) et (V) ou l'un de leurs sels avec les acides, notamment leurs chlorhydrates et leur trifluoroacétates : 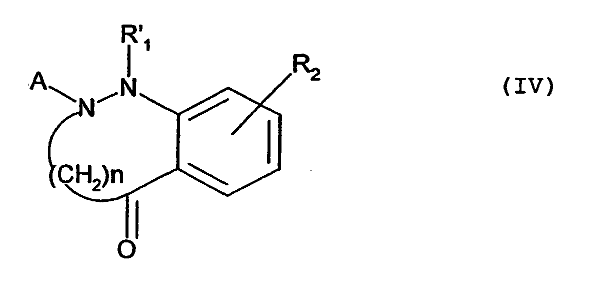 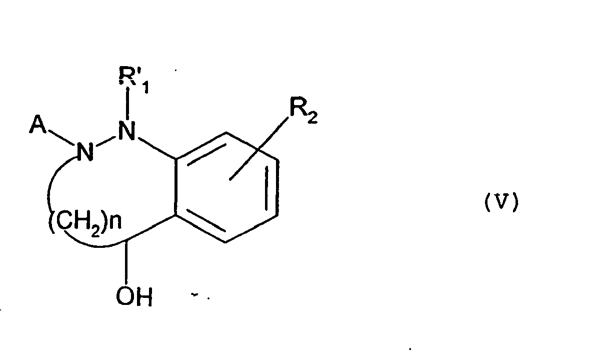
dans lesquelles A, R₂ et n sont définis comme à la revendication 12 et R'₁ représente un radical (CH₂)_{n'}R^{o}₁ dans lequel n' est égal à O ou 1 et R^{o}₁ représente un radical hétéroaryle renfermant jusqu'à 15 atomes de carbone et un ou plusieurs hétéroatomes choisis parmi les atomes d'azote, de soufre et d'oxygène, COR', CONR'R", CSNR'R", COCOOR', SO₂NR'R", SO₂R', CO₂R' ou CN,
R' représentant un atome d'hydrogène, un radical alkyle, alkényle renfermant jusqu'à 8 atomes de carbone, aralkyle renfermant jusqu'à 12 atomes de carbone ou aryle renfermant jusqu'à 12 atomes de carbone, et
R" représentant un atome d'hydrogène, un radical alkyle renfermant jusqu'à 8 atomes de carbone, aryl renfermant jusqu'à 12 atomes de carbone, aralkyle renfermant jusqu'à 12 atomes de carbone, SO₂-R'ou COR', R' étant défini comme ci-dessus.

20. Composé de formule (VI) ou l'un de ses sels avec un acide, notamment son chlorhydrate et son trifluoroacétate : 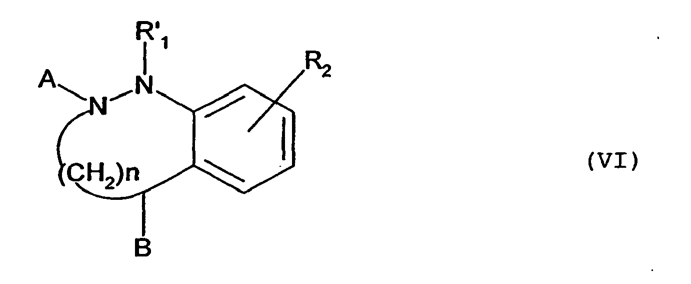 dans laquelle A, R'₁, R₂, B et n ont les mêmes significations qu'à la revendication 12.

21. Composés de formules (VII) et (VIII) ou l'un de leurs sels avec un acide, notamment leurs chlorhydrates et leurs trifluoroacétates : 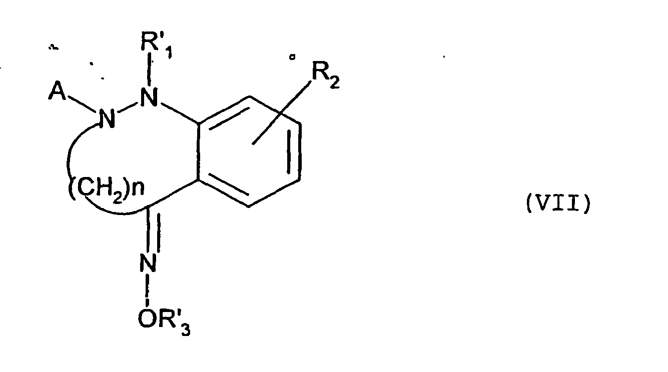 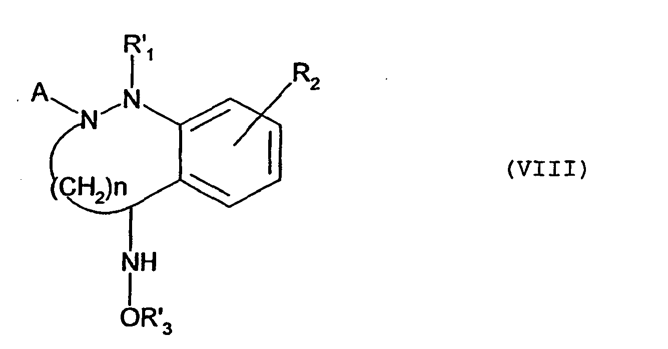 dans lesquelles A, R'₁, R₂, n et R'₃ sont définis comme à la revendication 13.

## Claims

1. A compound corresponding to the formula (I) or one of its salts with bases or acids: 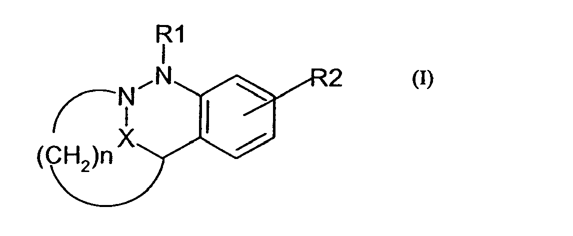 in which:
n is equal to 1 or 2,
R₁ represents a hydrogen atom, an alkyl radical including up to 8 carbon atoms or a (CH₂)_{n'}R^{o}₁ radical in which n' is equal to 0 or 1 and R^{o}₁ represents an aryl radical including up to 12 carbon atoms, an heteroaryl radical including up to 15 carbon atoms and one or more heteroatoms chosen from nitrogen, sulfur and oxygen atoms, a COR', CONR'R", CSNR'R", COCOOR', SO₂NR'R", SO₂R', CO₂R' or CN radical,
R' representing a hydrogen atom, an alkyl or alkenyl radical including up to 8 carbon atoms, an aralkyl radical including up to 12 carbon atoms or an aryl radical including up to 12 carbon atoms,
R" representing a hydrogen atom, an alkyl radical including up to 8 carbon atoms, an aryl radical including up to 12 carbon atoms, an aralkyl radical including up to 12 carbon atoms, an SO₂-R' radical or a COR' radical, R' being defined as above,
R₂ represents a hydrogen atom or one or more substituents chosen from the group consisting of the halo, alkyl, OH, Oalkyl, NO₂, NH₂, NHalkyl, N(alkyl)₂, NHCOalkyl, NHSO₂alkyl, CONHalkyl, SO₂NHalkyl, COOH, COOalkyl, CN, OSO₂alkyl, NHCONHalkyl and COalkyl radicals, alkyl including up to 8 carbon atoms,
X represents a divalent group -C(O)-N(OR₃)- connected to the nitrogen atom via the carbon atom, in which R₃ is chosen from the group consisting of a hydrogen atom and the R, Y, Y₁, Y₂ and Y₃ radicals,
R is chosen from the group consisting of an alkyl radical including up to 6 carbon atoms, optionally substituted by a pyridyl or carbamoyl radical, an alkenyl radical including up to 8 carbon atoms, an aryl radical including up to 12 carbon atoms or an aralkyl radical including up to 12 carbon atoms, the nucleus of the aryl or aralkyl radical optionally being substituted by an OH radical, an NH₂ radical, an NO₂ radical, an alkyl radical including up to 8 carbon atoms or an alkoxy radical including up to 8 carbon atoms or by one or more halogen atoms,
Y is chosen from the group consisting of the COR, COOH, COOR, CONHR, CONHOH, CONHSO₂R, CH₂COOH, CH₂COOR, CH₂CONHOH, CH₂CONHCN, CH₂tetrazole, CH₂(protected tetrazole), CH₂SO₃H, CH₂SO₂R, CH₂PO(OR)₂, CH₂PO(OR)(OH), CH₂PO(R)(OH) and CH₂PO(OH)₂ radicals,
Y₁ is chosen from the group consisting of the SO₂R, SO₂NHCOR, SO₂NHCOOR, SO₂NHCONHR and SO₃H radicals,
Y₂ is chosen from the group consisting of the PO(OH)₂, PO(OR)₂, PO(OH)(OR) and PO(OH)(R) radicals,
Y₃ is chosen from the group consisting of the following radicals: tetrazole, tetrazole substituted by the R radical, squarate, NRtetrazole, NRtetrazole substituted by the R radical and NRSO₂R, R being defined as above.

2. The compound as claimed in claim 1, wherein n is equal to 1.

3. The compound as claimed in claim 1 or claim 2, wherein R₂ represents a hydrogen atom.

4. The compound as claimed in one of claims 1 to 3, wherein R₁ represents a hydrogen atom, an alkyl radical including up to 8 carbon atoms or a (CH₂)_{n'}R^{o}₁ radical in which n' is equal to 0 or 1 and R^{o}₁ represents an aryl radical including up to 12 carbon atoms, an heteroaryl radical including up to 15 carbon atoms and one or more heteroatoms chosen from nitrogen, sulfur and oxygen atoms, and R' and R" being defmed as in claim 1.

5. The compound as claimed in one of claims 1 to 4, wherein X represents a divalent group -C(O)-N(OR₃)- in which R₃ is chosen from the group consisting of a hydrogen atom and the R, Y and Y₁ radicals, R, Y and Y₁ being defined as in claim 1.

6. The compounds of formula (I) as defined in claim 1, with the following names:
[[1,5-dihydro-1-(methylsulfonyl)-3-oxo-2,5-methano-2*H*-1,2,4-benzotriazepin-4(3*H*)-yl]oxy]acetic acid,
[[1-[(benzoylamino)carbonyl]-1,5-dihydro-3-oxo-2,5-methano-2*H*-1,2,4-benzotriazepin-4(3*H*)-yl]oxy]acetic acid,
[[1,5-dihydro-3-oxo-1-[(phenylsulfonyl)aminocarbonyl]-2,5-methano-2*H-*1,2,4-benzotriazepin-4(3*H*)-yl]oxy]acetic acid,
[(1,5-dihydro-3-oxo-2,5-methano-2*H*-1,2,4-benzotriazepin-4(3*H*)-yl)oxy]acetic acid,
4,5-dihydro-1-methyl-4-(sulfooxy)-2,5-methano-2*H*-1,2,4-benzotriazepin-3(1*H*)-one,
4,5-dihydro-4-(2-propenyloxy)-1-(3-pyridinylmethyl)-2,5-methano-2*H*-1,2,4-benzotriazepin-3(1*H*)one,
4,5-dihydro-3-oxo-*N*-(phenylsulfonyl)-4-(2-propenyloxy)-2,5-methano-2*H-*1,2,4-benzotriazepine-1(3*H*)-carboxamide,
*N*-benzoyl-4,5-dihydro-3-oxo-4-(2-propenyloxy)-2,5-methano-2*H*-1,2,4-benzotriazepine-1(3*H*)-carboxamide,
ethyl 4,5-dihydro-α,3-dioxo-4-(2-propenyloxy)-2,5-methano-2*H*-1,2,4-benzotriazepine-1(3*H*)-acetate,
ethyl 4,5-dihydro-3-oxo-4-(sulfooxy)-2,5-methano-2*H*-1,2,4-benzotriazepine-1(3*H*)-acetate,
and their salts as defined in claim 1.

7. A process for the preparation of a compound as claimed in one of claims 1 to 6, which comprises:
a) a stage during which a compound of formula (II): 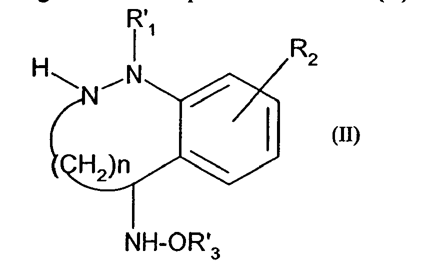 in which:
R'₁ represents an R₁ radical or a radical which is a precursor of R₁, R₂ and n are as defined in claim 1 and R'₃ represents a protective group for the hydroxyl or an Rp, Yp, Y₁p, Y₂p or Y₃p radical, these radicals respectively having the values of R, Y, Y₁, Y₂ and Y₃ indicated in claim 1, in which the possible reactive functional groups present are, if appropriate, protected, is reacted with a carbonylating agent, if appropriate in the presence of a base, for the purpose of obtaining an intermediate compound of formula (III): 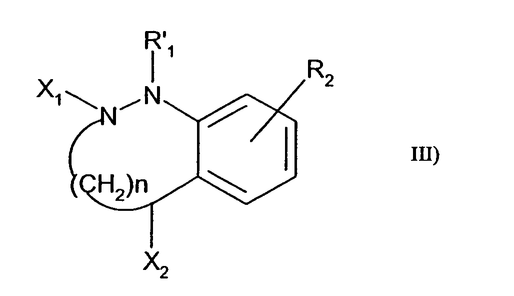
in which:
R'₁, R₂ and n have the same meanings as above and either X₁ is a hydrogen atom and X₂ represents an -N(OR'₃)-CO-X₃ group, R'₃ being as defined above and X₃ representing the residue of the carbonylating agent, or X₂ represents an -NH-OR'₃ group and X₁ represents a CO-X₃ group, X₃ being defined as above;
b) a stage during which the intermediate obtained above is cyclized, in the presence of a base;
and comprises:
c) if appropriate, preceding stage a) and/or succeeding stage b) by one or more of the following reactions, in an appropriate order:
- protection of the reactive functional groups,
- deprotection of the reactive functional groups,
- esterification,
- saponification,
- sulfonation,
- phosphatation,
- amidation,
- acylation,
- sulfonylation,
- alkylation,
- formation of a urea group,
- introduction of a tetrazole group,
- reduction of carboxylic acids,
- dehydration of amide to nitrile,
- salification,
- exchange of ions,
- separation of enantiomers,
- nitration,
- reduction of a nitro to an amino,
- halogenation,
- carbamoylation,
- introduction of a cyano group.

8. The process as claimed in claim 7, wherein the carbonylating agent is chosen from the group consisting of phosgene, diphosgene, triphosgene, aryl, aralkyl, alkyl and alkenyl chloroformates, alkyl dicarbonates, carbonyldiimidazole and their mixtures.

9. The process as claimed in claim 7 or claim 8, wherein the carbonylation reaction takes place in the presence of a base.

10. The process as claimed in one of claims 7 to 9, wherein, in stage b), the base is chosen from the group consisting of amines, alkali metal and alkaline earth metal, hydrides, alkoxides, amides and carbonates.

11. The process as claimed in claim 10, wherein the base is an amine.

12. The process as claimed in any one of claims 7 to 11, wherein the compound of formula (II) is obtained by a process according to which a compound of formula (IV): 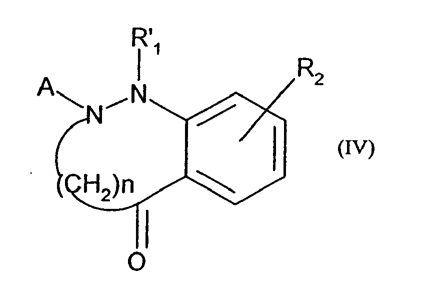 in which R'₁, R₂ and n are defined as in claim 7 and A represents a hydrogen atom or a protective group for the nitrogen, is treated with a reducing agent, to obtain a compound of formula (V): 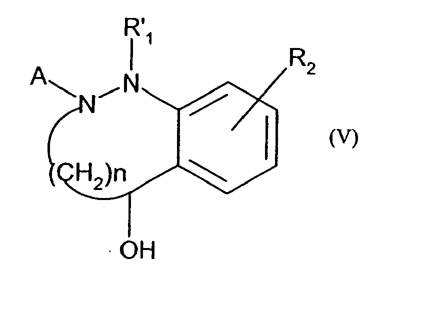 in which A, R'₁, R₂ and n retain their abovementioned meaning, in which, if appropriate, the OH group is replaced by a leaving group, to obtain a compound of formula (VI): 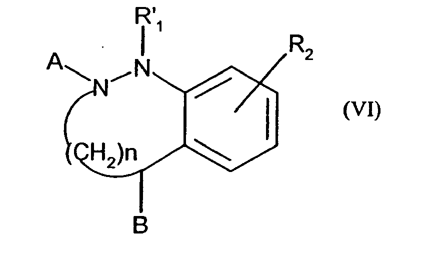 in which A, R'₁, R₂ and n retain their abovementioned meaning and B represents a leaving group, which is treated with a compound of formula NH₂-OR'₃, R'₃ retaining the meaning indicated in claim 7, and then, if appropriate, with an appropriate deprotecting agent for the nitrogen atom.

13. The process as claimed in any one of claims 7 to 11, wherein the compound of formula (II) is obtained by a process according to which a compound of formula (IV) as defined in claim 12 is treated with a compound of formula H₂NOR'₃, to obtain a compound of formula (VII): 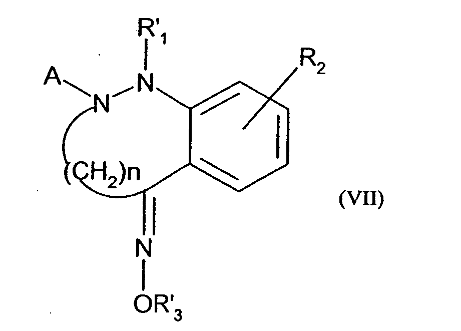 in which A is defined as in claim 12 and R'₁, R₂, n and R'₃ are defined as in claim 7, which is reacted with a reducing agent, to obtain a compound of formula (VIII): 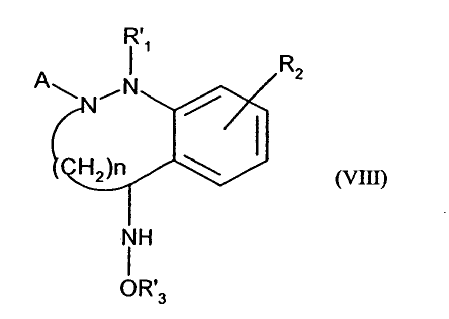 in which A, R'₁, R₂, n and R'₃ are defined as above, which is treated, if appropriate, with an appropriate deprotecting agent for the nitrogen atom.

14. As medicaments, the products as defined in any one of claims 1 to 5 and their salts with pharmaceutically acceptable acids and bases.

15. As medicaments, the products as defined in claim 6 and their salts with pharmaceutically acceptable acids and bases.

16. Pharmaceutical compositions comprising, as active principle, at least one medicament as claimed in either of claims 14 and 15.

17. A compound of general formula (III) or one of its salts with an acid, in particular its hydrochloride and its trifluoroacetate: 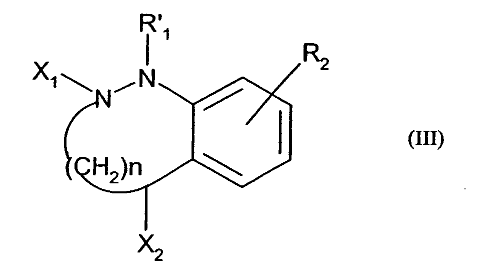 in which:
R'₁, R₂, X₁, X₂ and n have the same meanings as in claim 7.

18. A compound of general formula (II) or one of its salts with an acid, in particular its hydrochloride and its trifluoroacetate: 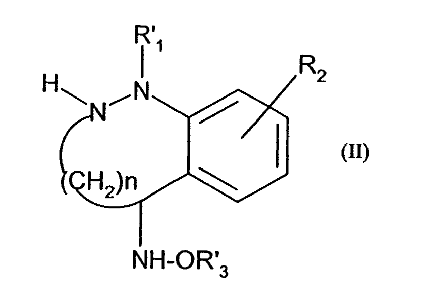 in which R'₁, R₂, R'₃ and n have the same meanings as in claim 7.

19. Compounds of formulae (IV) and (V) or one of their salts with acids, in particular their hydrochlorides and their trifluoroacetates: 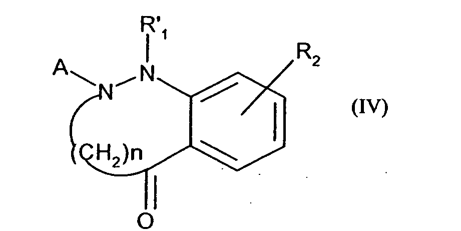 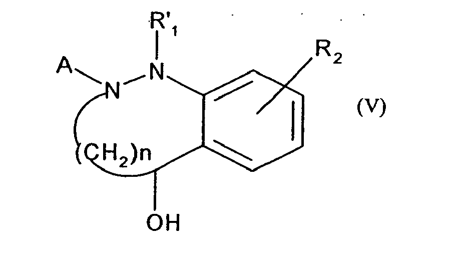
in which A, R₂ and n have the same meanings as in claim 12 and R'₁ represents a radical (CH₂)n'R)^{o}₁ in which n' is 0 or 1 nd R^{o}₁ represents an heteroaryl radical containing up to 15 carbon atoms and one or more heteroatoms selected from nitrogen, sulfur and oxygen, COR', CONR'R", CSNR'R", COCOOR', SO₂NR'R", SO₂R', CO₂R' or CN,
R' represents a hydrogen atom, an alkyl or alkenyl radical containing up to 8 carbon atoms, aralkyl containing up to 12 carbon atoms or aryl containing up to 12 carbon atoms, and
R" represents a hydrogen atom, an alkyl radical containing up to 8 carbon atoms, an aryl radical containing up to 12 carbon atoms, an aralkyl radical containing up to 12 carbon atoms, SO₂-R' or COR', R' being defined as above.

20. A compound of formula (VI) or one of its salts with an acid, in particular its hydrochloride and its trifluoroacetate: 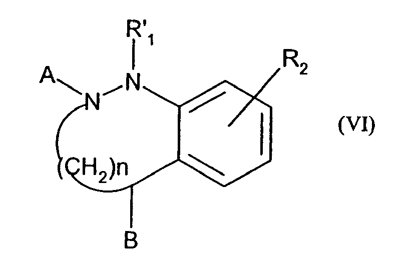 in which A, R'₁, R₂, B and n have the same meanings as in claim 12.

21. Compounds of formulae (VII) and (VIII) or one of their salts with an acid, in particular their hydrochlorides and their trifluoroacetates: 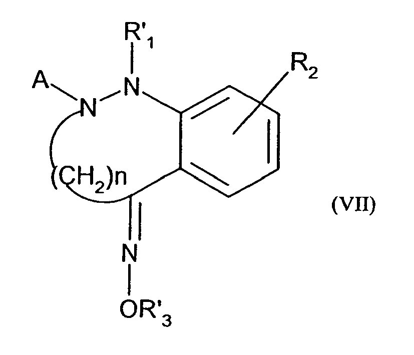 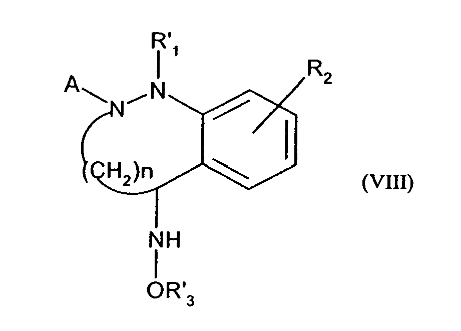 in which A, R'₁, R₂, n and R"₃ are defined as in claim 13.

## Patentansprüche

1. Verbindung mit der Formel (I) oder eines ihrer Salze von Basen oder Säuren: 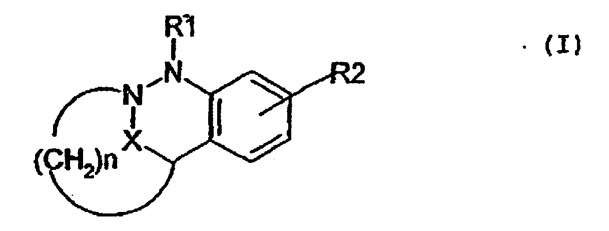 wobei:
n gleich 1 oder 2 ist,
R₁ ein Wasserstoffatom darstellt oder ein Alkylradikal, das bis zu 8 C-Atome umfasst, ein (CH₂)_{n'}R^{o}₁-Radikal, bei dem n' gleich 0 oder 1 ist, und R^{o}₁ ein Arylradikal, das bis zu 12 C-Atome umfasst, oder ein Heteroarylradikal darstellt, das bis zu 15 C-Atome und ein Heteroatom oder mehrere Heteroatome umfasst, die aus der aus den Stickstoff-, Schwefel- oder Sauerstoffatomen, COR', CONR'R", CSNR'R", COCOOR', SO₂NR'R", SO₂R', CO₂R' oder CN bestehenden Gruppe ausgewählt sind,
R' ein Wasserstoffatom, ein Alkylradikal, Alkenylradikal, das bis zu 8 C-Atome umfasst, Aralkylradikal, das bis zu 12 C-Atome umfasst, oder Arylradikal, das bis zu 12 C-Atome umfasst, darstellt,
R" ein Wasserstoffatom, ein Alkylradikal, das bis zu 8 C-Atome umfasst, ein Arylradikal, das bis zu 12 C-Atome umfasst, ein Aralkylradikal, das bis zu 12 C-Atome umfasst, SO₂-R' oder COR' darstellt, wobei R' wie oben definiert ist,
R₂ ein Wasserstoffatom oder einen oder mehrere Substituenten darstellt, die aus der aus den Halogen-, Alkyl-, OH-, Oalkyl-, NO₂-, NH₂-, NHalkyl-, N(Alkyl)₂-, NHCOalkyl-, NHSO₂alkyl-, CONHalkyl-, SO₂NHalkyl-, COOH-, COOalkyl-, CN-, OSO₂alkyl-, NHCONHalkyl- und COalkyl-Radikalen bestehenden Gruppe ausgewählt sind, wobei das Alkyl bis zu 8 C-Atome umfasst,
X eine bivalente -C(O)-N(OR₃)-Gruppe darstellt, die mit dem Stickstoffatom durch das C-Atom verbunden ist, wobei R₃ aus der aus einem Wasserstoffatom und den Radikalen R, Y, Y₁, Y₂ und Y₃ bestehenden Gruppe ausgewählt ist,
R aus der Gruppe ausgewählt ist, die aus einem Alkylradikal, das bis zu 6 C-Atome umfasst, das eventuell substituiert ist durch ein Pyridyl- oder Carbamoylradikal, ein Alkenylradikal, das bis zu 8 C-Atome umfasst, ein Arylradikal, das bis zu 12 C-Atome umfasst oder Aralkylradikal besteht, das bis zu 12 C-Atome umfasst, wobei der Kern des Aryl- oder Aralkylradikals eventuell durch ein OH-, NH₂-, NO₂-, Alkyl das bis zu 8 C-Atome umfasst, Alkoxy das bis zu 8 C-Atome umfasst, oder durch ein Halogenatom oder mehrere Halogenatome substituiert ist,
Y aus der Gruppe ausgewählt ist, die aus den Radikalen COR, COOH, COOR, CONHR, CONHOH, CONHSO₂R, CH₂COOH, CH₂COOR, CH₂CONHOH, CH₂CONHCN, CH₂Tetrazol, geschütztes CH₂Tetrazol, CH₂SO₃H, CH₂SO₂R, CH₂PO(OR)₂, CH₂PO (OR)(OH), CH₂PO(R)(OH) und CH₂PO(OH)₂ gebildet ist,
Y₁ aus der Gruppe ausgewählt ist, die aus den Radikalen SO₂R, SO₂NHCOR, SO₂NHCOOR, SO₂NHCONHR und SO₃H gebildet ist,
Y₂ aus der Gruppe ausgewählt ist, die aus den Radikalen PO(OH)₂, PO(OR)₂, PO(OH)(OR) und PO(OH)(R) gebildet ist,
Y₃ aus der Gruppe ausgewählt ist, die aus den Radikalen Tetrazol, durch das Radikal R substituiertes Tetrazol, Squarat, NR Tetrazol, durch das Radikal R substituiertes NR Tetrazol und NRSO₂R gebildet ist, wobei R wie oben definiert ist.

2. Verbindung nach Anspruch 1, **dadurch gekennzeichnet, dass** n gleich 1 ist.

3. Verbindung nach Anspruch 1 oder Anspruch 2, **dadurch gekennzeichnet, dass** R₂ ein Wasserstoffatom darstellt.

4. Verbindung nach einem der Ansprüche 1 bis 3, **dadurch gekennzeichnet, dass** R₁ ein Wasserstoffatom, ein Alkylradikal, das bis zu 8 C-Atome umfasst, ein (CH₂)_{n'}R^{o}₁-Radikal, wobei n' gleich 0 oder 1 ist, darstellt und R^{o}₁ ein Arylradikal, das bis zu 12 C-Atome umfasst, oder ein Heteroaryl darstellt, das bis zu 15 C-Atome und ein Heteroatom oder mehrere Heteroatome umfasst, die aus der aus Stickstoff-, Schwefel- und Sauerstoffatomen, CONR'R", CSNR'R", COCOOR', SO₂NR'R", SO₂R' oder CO₂R' bestehenden Gruppe ausgewählt sind, wobei R' und R" wie in Anspruch 1 definiert sind.

5. Verbindung nach einem der Ansprüche 1 bis 4, **dadurch gekennzeichnet, dass** X eine bivalente -C(O)-N(OR₃)-Gruppe darstellt, wobei R₃ aus der aus einem Wasserstoffatom und den Radikalen R, Y, Y₁ bestehenden Gruppe ausgewählt ist, wobei R, Y und Y₁ wie in Anspruch 1 definiert sind.

6. Verbindung der Formel (I) wie in Anspruch 1 definiert, deren Bezeichnungen folgen:
- [[1,5-Dihydro-1-(Methylsulfonyl)-3-oxo-2,5-Methan-2*H*-1,2,4-Benzotriazepin-4(3*H*)-yl]oxy]Essigsäure,
- [[1-[(Benzoylamino)Carbonyl]-1,5-Dihydro-3-oxo-2,5-Methan-2*H*-1,2,4-Benzotriazepin-4(3*H*)-yl]oxy]Essigsäure,
- [[1,5-Dihydro-3-oxo-1-(Phenylsulfonyl)Aminocarbonyl] -2,5-Methan-2*H*-1,2,4-Benzotriazepin-4(3*H*)-yl]oxy] Essigsäure,
- [[1,5-Dihydro-3-oxo-2,5-Methan-2*H*-1,2,4-Benzotriazepin-4(3*H*)-yl]oxy]Essigsäure,
- 4,5-Dihydro-1-Methyl-4-(Sulfooxy)-2,5-Methan-2*H-*1,2,4-Benzotriazepin-3(1*H*)-on,
- 4,5-Dihydro-4-(2-Propenyloxy)-1-(3-Pyridinylmethyl)-2,5-Methan-2*H*-1,2,4-Benzotriazepin-3(1*H*)-on,
- 4,5-Dihydro-3-oxo-N-(Phenylsulfonyl)-4-(2-Propenyloxy)-2,5-Methan-2*H*-1,2,4-Benzotriazepin-1(3*H*)-Carboxamid,
- N-Benzoyl-4,5-Dihydro-3-oxo-4-(2-Propenyloxy)-2,5-Methan-2*H*-1,2,4-Benzotriazepin-1(3*H*)-Carboxamid,
- 4,5-Dihydro-α,3-Dioxo-4-(2-Propenyloxy)-2,5-Methan-2*H*-1,2,4-Benzotriazepin-1(3*H*)-Ethylazetat,
- 4,5-Dihydro-3-oxo-4-(Sulfooxy)-2,5-Methan-2*H*-1,2,4-Benzotriazepin-1(3*H*)-Ethylazetat,
sowie ihre Salze wie in Anspruch 1 definiert.

7. Verfahren zur Zubereitung einer Verbindung nach einem der Ansprüche 1 bis 6, **dadurch gekennzeichnet, dass** es umfasst:
a) einen Schritt, in dessen Verlaufe man mit einem Carbonylierungsmittel, gegebenenfalls in Gegenwart einer Base, die Verbindung der Formel (II) reagieren lässt: 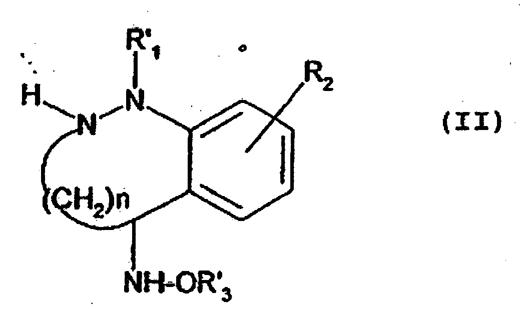 wobei
R'₁ ein R₁-Radikal darstellt oder ein Radikal, das ein Vorläufer von R₁ ist, R₂ und n wie in Anspruch 1 definiert sind und R'₃ eine schützende Gruppe des Hydroxy oder ein Radikal Rp, Yp, Y₁p, Y₂p oder Y₃p darstellt, wobei diese Radikale jeweils die Werte von R, Y, Y₁, Y₂ und Y₃ haben, die in Anspruch 1 angegeben sind, in denen die eventuellen vorhandenen Reaktionsfunktionen gegebenenfalls geschützt sind, um eine Zwischenverbindung der Formel (III) zu erhalten: 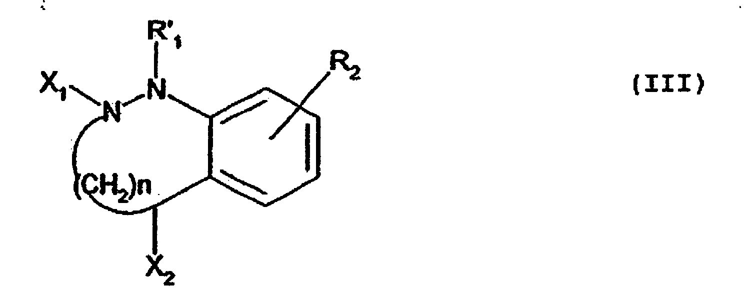
wobei
R'₁, R₂ und n dieselben Bedeutungen wie oben haben und entweder X₁ ein Wasserstoff ist und X₂ eine -N(OR'₃)-CO-X₃-Gruppe, wobei R'₃ wie oben definiert ist, und X₃ den Rest des Carbonylierungsmittels darstellen, oder X₂ eine -NH-OR'₃-Gruppe und X₁ eine CO-X₃-Gruppe darstellen, wobei X₃ wie oben definiert ist;
b) einen Schritt, in dessen Verlaufe man die vorher erhaltene Zwischenverbindung in Gegenwart einer Base zyklisiert;
und dass
c) gegebenenfalls dem Schritt a) eine oder mehrere der folgenden Reaktionen voraus geht oder gehen und/oder diese dem Schritt b) folgt oder folgen und zwar in geeigneter Reihenfolge:
- Schützen der Reaktionsfunktionen,
- Entschützen der Reaktionsfunktionen,
- Veresterung,
- Verseifung,
- Sulfatierung,
- Phosphatierung,
- Amidifizierung,
- Acylierung,
- Sulfonylierung,
- Alkylierung,
- Bildung einer Harnstoffgruppe,
- Einführung einer Tetrazolgruppe,
- Reduktion von Carbonsäuren,
- Dehydratisierung eines Amids zu einem Nitril,
- Salzbildung,
- Ionenaustausch,
- Trennung von Enantiomeren,
- Nitrierung,
- Reduktion eines Nitro zu einem Amin,
- Halogenierung,
- Carbamoylierung,
- Zyanidlaugung.

8. Verfahren nach Anspruch 7, **dadurch gekennzeichnet, dass** das Carbonylisierungsmittel aus der aus Phosgen, Diphosgen, Triphosgen, den Aryl-, Aralkyl-, Alkyl- und Alkenylchlorformiaten, Alkyldicarbonaten, dem Carbonyldiimidazol und ihren Gemischen bestehenden Gruppe ausgewählt ist.

9. Verfahren nach Anspruch 7 oder Anspruch 8, **dadurch gekennzeichnet, dass** die Carbonylisierungsreaktion in Gegenwart einer Base stattfindet.

10. Verfahren nach einem der Ansprüche 7 bis 9, **dadurch gekennzeichnet, dass** beim Schritt b) die Base aus der aus Aminen, Hydriden, Alkoholaten, Amiden oder Carbonaten der Alkalimetalle oder Erdalkalimetalle bestehenden Gruppe ausgewählt ist.

11. Verfahren nach Anspruch 10, **dadurch gekennzeichnet, dass** die Base ein Amin ist.

12. Verfahren nach irgendeinem der Ansprüche 7 bis 11, **dadurch gekennzeichnet, dass** die Verbindung der Formel (II) durch ein Verfahren erhalten wird, nach dem man eine Verbindung der Formel (IV): 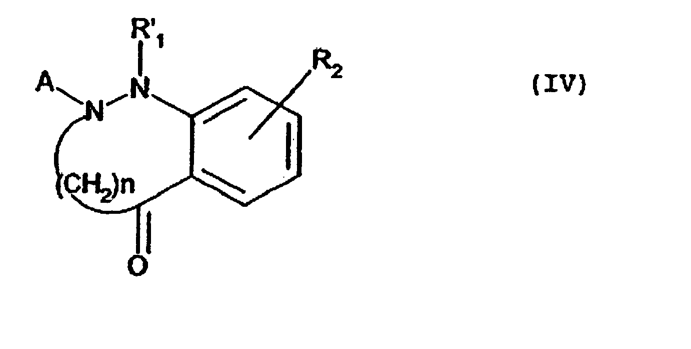 wobei R'₁, R₂ und n wie in Anspruch 7 definiert sind und A ein Wasserstoffatom oder eine schützende Gruppe des Stickstoffs darstellt, mit einem Reduktionsmittel behandelt, um eine Verbindung der Formel (V) zu erhalten: 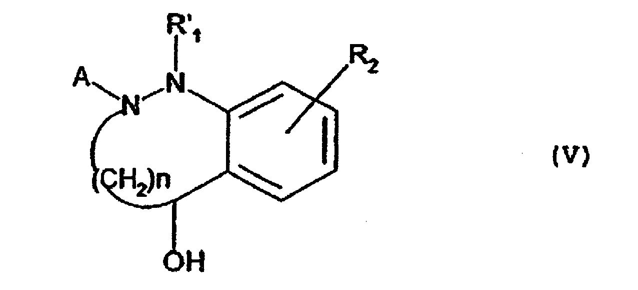 wobei A, R'₁, R₂ und n ihre oben genannte Bedeutung behalten, wobei man gegebenenfalls die OH-Gruppe durch eine Abgangsgruppe ersetzt, um eine Verbindung der Formel (VI) zu erhalten: 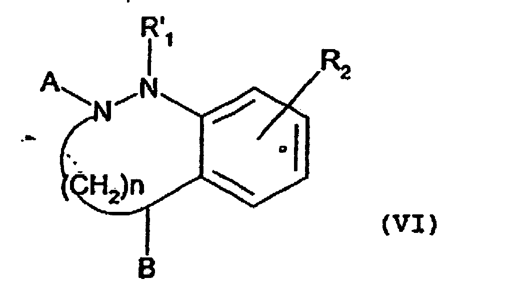 wobei A, R'₁, R₂ und n ihre oben genannte Bedeutung behalten und B eine Abgangsgruppe darstellt, die man mit einer Verbindung der Formel NH₂-OR'₃, wobei R'₃ die in Anspruch 7 angegebene Bedeutung behält, dann gegebenenfalls mit einem geeigneten entschützenden Mittel des Stickstoffatoms behandelt.

13. Verfahren nach irgendeinem der Ansprüche 7 bis 11, **dadurch gekennzeichnet, dass** die Verbindung der Formel (II) durch ein Verfahren erhalten wird, nach dem man eine Verbindung der Formel (IV), wie sie in Anspruch 12 definiert ist, mit einer Verbindung der Formel H₂N-OR'₃ behandelt, um eine Verbindung der Formel (VII) zu erhalten: 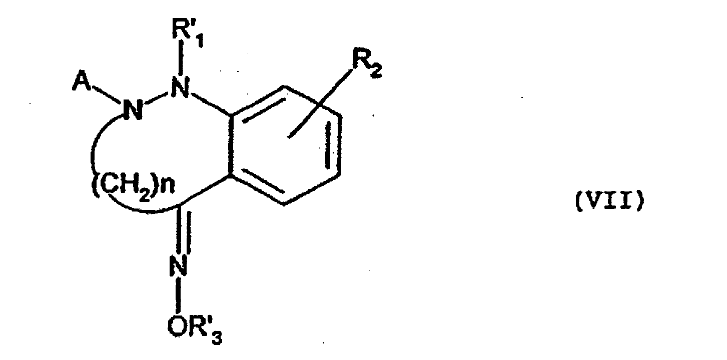 wobei A wie in Anspruch 12 definiert ist und R'₁, R₂, n und R'₃ wie in Anspruch 7 definiert sind, die man mit einem Reduktionsmittel reagieren lässt, um eine Verbindung der Formel (VIII) zu erhalten: 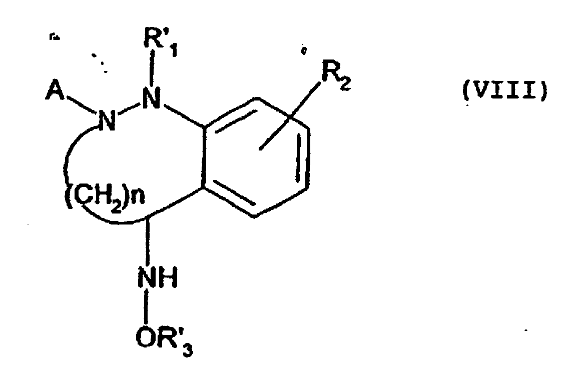 wobei A, R'₁, R₂, n und R'₃ wie oben definiert sind, die man gegebenenfalls mit einem geeigneten Entschützungsmittel des Stickstoffatoms behandelt.

14. Produkte, die in irgendeinem der Ansprüche 1 bis 5 definiert sind, sowie ihre Salze mit pharmazeutisch annehmbaren Säuren und Basen als Medikamente.

15. Produkte, die in Anspruch 6 definiert sind, sowie ihre Salze mit pharmazeutisch annehmbaren Säuren und Basen als Medikamente.

16. Pharmazeutische Zusammensetzungen, die als Wirkstoff mindestens ein Medikament nach einem der Ansprüche 14 oder 15 enthalten.

17. Zusammensetzung der allgemeinen Formel (III) oder eines ihrer Salze mit einer Säure, insbesondere ihr Chlorhydrat und ihr Trifluoracetat: 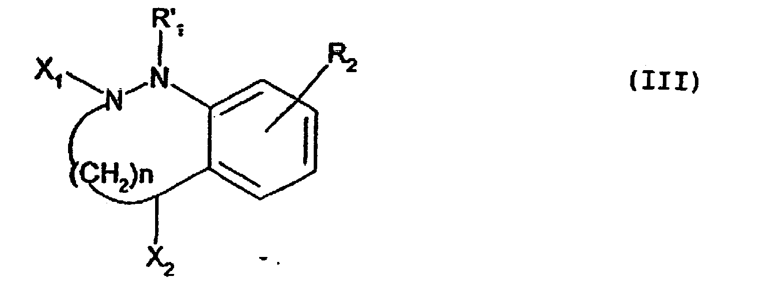 wobei
R'₁, R₂, X₁, X₂ und n dieselben Bedeutungen wie in Anspruch 7 haben.

18. Zusammensetzung der allgemeinen Formel (II) oder eines ihre Salze mit einer Säure, insbesondere ihr Chlorhydrat und ihr Trifluoracetat: 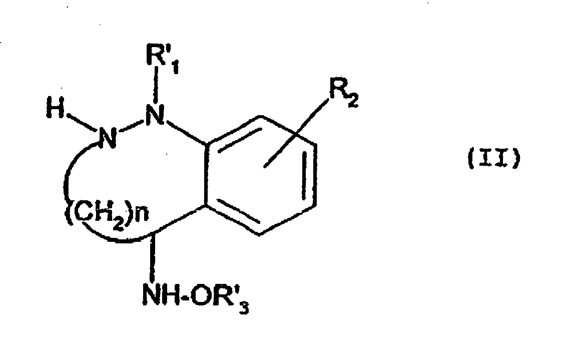 wobei R'₁, R₂, R'₃ und n dieselben Bedeutungen wie in Anspruch 7 haben.

19. Zusammensetzung der Formel (IV) und (V) oder eines ihrer Salze mit Säuren, insbesondere ihre Chlorhydrate und ihre Trifluoracetate: 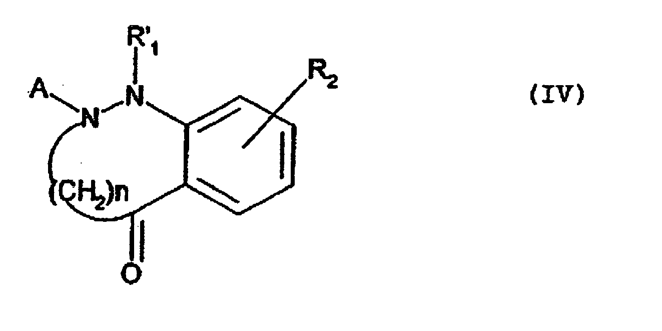 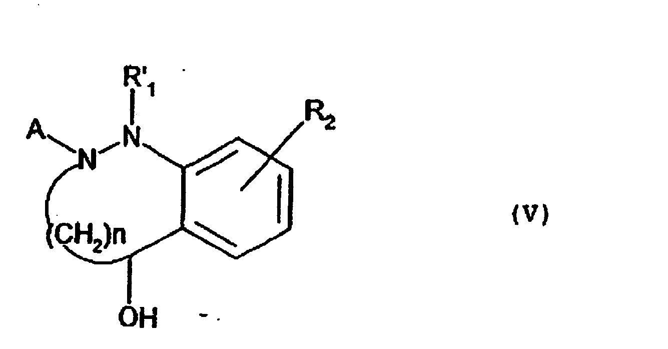
wobei A, R₂ und n wie in Anspruch 12 definiert sind, und R'₁ ein (CH₂)_{n'}R^{o}₁-Radikal darstellt, wobei n' gleich 0 oder 1 ist und R^{o}₁ ein Heteroarylradikal darstellt, das bis zu 15 C-Atome und ein Heteroatom oder mehrere Heteroatome umfasst, die aus der aus den Stickstoff-, Schwefel- oder Sauerstoffatomen, COR', CONR'R", CSNR'R", COCOOR', SO₂NR'R", SO₂R', CO₂R' oder CN bestehenden Gruppe ausgewählt sind,
R' ein Wasserstoffatom, ein Alkylradikal, ein Alkenylradikal, das bis zu 8 C-Atome umfasst, ein Aralkylradikal, das bis zu 12 C-Atome umfasst, oder ein Arylradikal, das bis zu 12 C-Atome umfasst, darstellt,und
R" ein Wasserstoffatom, ein Alkylradikal, das bis zu 8 C-Atome umfasst, ein Arylradikal, das bis zu 12 C-Atome umfasst, ein Aralkylradikal, das bis zu 12 C-Atome umfasst, SO₂-R' oder COR' darstellt, wobei R' wie oben definiert ist.

20. Zusammensetzung der Formel (VI) oder eines ihre Salze mit einer Säure, insbesondere ihr Chlorhydrat und ihr Trifluoracetat: 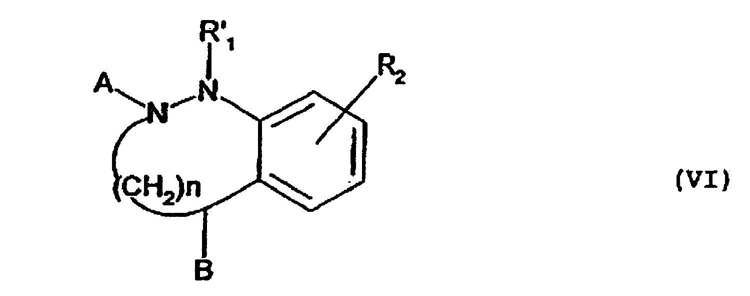 wobei A, R'₁, R₂, B und n dieselben Bedeutungen wie in Anspruch 12 haben.

21. Zusammensetzung der Formel (VII) und (VIII) oder eines ihre Salze mit einer Säure, insbesondere ihre Chlorhydrate und ihre Trifluoracetate: 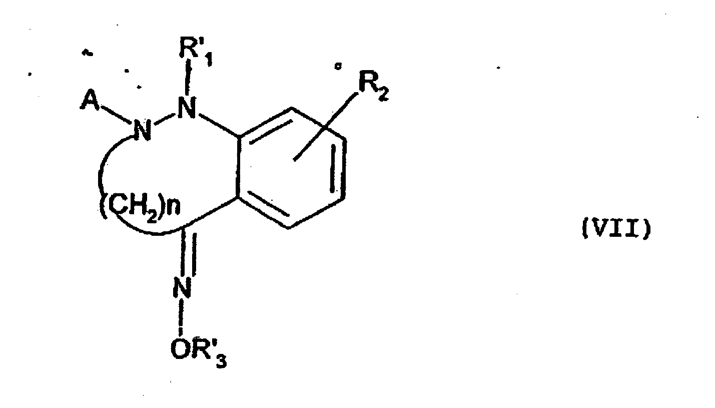 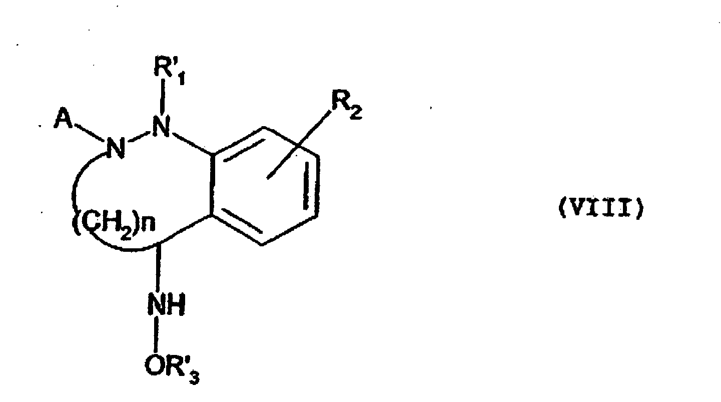 wobei A, R'₁, R₂, n und R'₃ wie in Anspruch 13 definiert sind.
